# EUROPEAN PATENT APPLICATION

(11) **EP 4 483 907 A1**
(43) Date of publication of application: **01.01.2025**
(21) Application number: 23760410.3
(22) Date of filing: 23.02.2023
(51) Int. Cl.: A61K 47/68, A61K 47/60, A61K 47/10, A61K 47/65, A61K 38/05

(54) **NOVEL ANTIBODY DRUG CONJUGATE**

(30) Priority: 25.02.2022 KR 20220025197
(71) Applicant: ABTIS Co., Ltd., Gyeonggi-do 16648 (KR); Research Business Foundation Sungkyunkwan University, Suwon-si, Gyeonggi-do 16419 (KR)
(72) Inventor: CHUNG, Sang Jeon, Suwon-si Gyeonggi-do 16420 (KR); OH, Yeong Soo, Gwacheon-si Gyeonggi-do 13833 (KR); KIM, Ju Hwan, Suwon-si Gyeonggi-do 16489 (KR); LEE, Sun Min, Hwaseong-si Gyeonggi-do 18337 (KR); LEE, Tae Jin, Suwon-si Gyeonggi-do 16627 (KR); LEE, Young Geun, Icheon-si Gyeonggi-do 17309 (KR); SEO, Jin Woo, Suwon-si Gyeonggi-do 16408 (KR); LEE, Geon Min, Hwaseong-si Gyeonggi-do 18264 (KR)
(74) Representative: Schön, Christoph
(86) International application number: PCT/KR2023/002629
(87) International publication number: WO 2023/163536

(57) **Abstract**

The present invention relates to a novel antibody drug conjugate and, specifically, to an antibody drug conjugate comprising polyethylene glycol, a solvate thereof, a pharmaceutically acceptable salt thereof, or a pharmaceutical composition containing the same, wherein the conjugate has excellent physical properties and stability.

## Description

### [Technical Field]

The present invention relates to a novel antibody-drug conjugate, and more particularly, to an antibody-drug conjugate comprising a polyethylene glycol unit, a solvate thereof, a pharmaceutically acceptable salt thereof, or a pharmaceutical composition comprising the same.

### [Background Art]

An antibody-drug conjugate (ADC) is a compound consisting of an antibody, a drug or payload, and a linker, and is prepared by covalently bonding an antibody binding to a specific target antigen with a drug exhibiting a pharmacological effect such as an apoptotic effect. ADCs require targeting ability to selectively recognize an antigen or receptor, the ability to induce apoptosis of specific cells, and linker stabilization and dissociation ability, and the antibody-drug conjugate satisfying these requirements may selectively act only on specific cells through targeting, thereby reducing side reactions and also increasing a therapeutic effect. The research and development of antibody-drug conjugates is progressing in various ways and is growing rapidly, and drug conjugates such as Adcetris, Kadcyla, Besponsa, Mylotarg, Polivy, and Padcev have been approved by the FDA.

Polyethylene glycol (PEG) is a water-soluble, biocompatible and nontoxic polymer having an ethylene oxide monomer as a unit, and used in the pharmaceutical field to extend plasma half-life, prevent enzymatic degradation, and provide sustained release through PEGylation. There have also been attempts in the antibody-drug conjugate field to provide beneficial effects using polyethylene glycol. In this regard, in US Patent No. 11,103,593, a conjugate comprising PEG unit is disclosed, and PEGylation for increasing antitumor activity and improving stability was disclosed. However, although PEGylation is performed, drug properties are not improved in all antibody-drug conjugates, and the effects of the conjugates may vary depending on a linker, a drug, etc. Accordingly, there is still a demand for antibody-drug conjugates with high stability and improved physical properties.

### Related Art Documents

(Patent Document 1) US Patent No. 1110359
(Non-Patent Document 1) Construction of paclitaxel-based antibody-drug conjugates with a PEGylated linker to achieve superior therapeutic index, Ting Shao et al., Signal Transduction and Targeted Therapy 5, 2020
(Non-Patent Document 2) Optimization of a PEGylated Glucuronide-Monomethylauristatin E Linker for Antibody-Drug Conjugates, Patrick J Burke et al., Mol Cancer Ther, 2017
(Non-Patent Document 3) An optimal "Click" formulation strategy for antibody-drug conjugate synthesis, Erol C Vtansever et al., Bioorg Med Chem, 2020
(Non-Patent Document 4) Click Chemistry Conjugations, Tak Ian Chio et al., Methods Mol Biol, 2020

### [Disclosure]

### [Technical Problem]

The present invention is directed to providing a novel linker that can exhibit excellent properties, and prevent a hydrophobic drug from precipitating.

The present invention is also directed to providing an antibody-drug conjugate (ADC) comprising the linker, or a pharmaceutically acceptable salt thereof.

The present invention is also directed to providing a pharmaceutical composition that comprises the novel ADC.

### [Technical Solution]

Hereinafter, the present invention will be described in detail.

The present invention provides a conjugate represented by Formula 1 below. 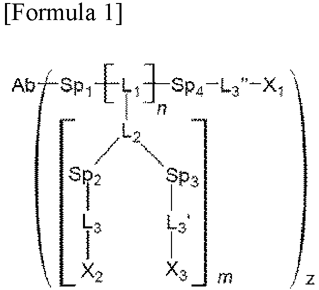 wherein,
Ab is a ligand,
Sp₁, Sp₂, Sp₃ and Sp₄ are each independently a spacer,
L₁ is a first linker unit,
L₂ is a second linker unit,
L₃, L₃' and L₃" are each independently selected third linker unit,
X₁, X₂, and X₃ are each independently selected from active agent or polyethylene glycol unit,
herein, at least one of X₁, X₂, and X₃ are polyethylene glycol unit,
z is an integer from 1 to 20,
n is an integer from 1 to 20,
m is an integer from 1 to 20,
wherein, when both n and m are 2 or more, L₁, L₂, L₃, L₃', Sp₂, Sp₃, X₂ and X₃ are each independently selected.

The present invention comprises a solvate of the conjugate or a pharmaceutically acceptable salt thereof.

In one embodiment of the present invention, the Ab is an antibody, an antibody fragment, or an antigen-binding fragment thereof.

In the present invention, the antibody, the antibody fragment, or the antigen-binding fragment thereof is a monoclonal antibody, a polyclonal antibody, an antibody fragment, Fab, Fab', F(ab)₂, Fv, scFv, a diabody, a linear antibody, a bispecific antibody, a multispecific antibody, a chimeric antibody, a humanized antibody, a fully human antibody, or an antigen-binding fragment thereof. In addition, in the present invention, the antibody, the antibody fragment, or the antigen-binding fragment may comprise a region of a light chain (V_{L} region), a region of heavy chain (V_{H} region), a constant domain thereof, or a variable domain thereof. In addition, the present invention comprises variants of the antibody, which are variants in which the rate of one or more of the affinity/binding force/specificity/selectivity of the antibody is 50% or more, 60% or more, 70% or more, 80% or more, 90% or more, 91% or more, 92% or more, 93% or more, 94% or more, 95% or more, 96% or more, or 97% or more.

Non-limiting examples of the antibodies comprise IgA, IgG, IgG1, IgG2, IgG3, IgG4, IgE, and IgD in the present invention.

Non-limiting examples of the antibodies in the present invention are as follows:
anti-CD71 antibody, anti-EGFR antibody, anti-HER2 antibody, anti-CD20 antibody, anti-CA125 antibody, anti-CD19 antibody, anti-CD44 antibody, anti-CD22 antibody, anti-FAP antibody, anti-CD38 antibody, anti-CD56 antibody, anti-CEA antibody, anti-CD2 antibody, anti-CD3 antibody, anti-CD7 antibody, anti-CD10 antibody, anti-CD167 antibody, anti-CD140 antibody, anti-CD121 antibody, anti-CD145 antibody, anti-CD71 antibody, CCK2R, CLDN18 (Claudin-18), CRIPTO, CSF2, CTLA4, DLL3, DLL4, interferon, interleukins (IL-1, IL-2, IL4, IL-9, etc.), IFN, NGF,
cetuximab, panitumumab, nimotuzumab, brentuximab, rituximab, ofatumumab, tositumomab, iburitumomab, trastuzumab, pertuzumab, ozogamicin, ozogamicin, ipilimumba, labetuzumab, lintuzumab, matuzumab, ciplizumab, sontuzumab, mepolizumab, motavizumab, motobizumab, natalizumab, nolovizumab, numavizumab, omalizumab, palvizumab, pascolizumab, pecfusituzumab, pectuzumab, pertuzumab, pexelizumab, ranibizumab, reslivisumab, reslizumab, resyvizumab, rovelizumab, ruplizumab, sibrotuzum, tacatuzumab, tetraxetan, tadocizumab, talizumab, tefibazumab, tocilizumab, toralizumab, trastuzumab, tucusituzumab, urtoxazumab, vislizumab, CNTO 95, huC242, and huN901.

In one embodiment of the present invention, a linker expressed as a combination of some of Sp₁, Sp₂, Sp₃, Sp₄, L₁, L₂, L₃, L₃', L₃", X₁, X₂, and X₃ is a cleavable linker or a non-cleavable linker.

In one embodiment of the present invention, the linker is a cleavable linker.

In one embodiment of the present invention, the cleavable linker may be protease-sensitive, pH-sensitive, or glutathione-sensitive.

In one embodiment of the present invention, the linker may be an enzyme-cleavable linker. The linker that can be cleaved by an enzyme may comprise one or two or more sites.

In an exemplary embodiment of the present invention, the linker represented by a combination of some of Sp₁, Sp₂, Sp₃, Sp₄, L₁, L₂, L₃, L₃', L₃", X₁, X₂, and X₃ is a cleavable linker that can be cleaved by glucuronidase. In addition, the linker is a linker that can be cleaved by β-glucuronidase.

In addition, in an exemplary embodiment of the present invention, the linker expressed as a combination of some of Sp₁, Sp₂, Sp₃, Sp₄, L₁, L₂, L₃, L₃', L₃", X₁, X₂, and X₃ is a cleavable linker that can be cleaved by cathepsin. For example, the linker may comprise a valine-citrulline (val-cit), lysine-lysine (lys-lys), phenylalanine-lysine (phe-lys), valine-alanine (Val-Ala) or alanine-alanine (Ala-Ala) linker and the like. In addition, the cleavable linker can be cleaved by cathepsin and also maintain a stable state *in vivo.* In addition, in a specific example, the cleavable linker can be cleaved by cathepsin B, cathepsin C, or cathepsin D, and also maintain a stable state *in vivo.* Among these linkers, the valine-citrulline (Val-Cit) linker is a linker that can be cleaved by cathepsin B.

In addition, in the linker expressed as a combination of some of Sp₁, Sp₂, Sp₃, Sp₄, L₁, L₂, L₃, L₃', L₃", X₁, X₂, and X₃ in the present invention, some of a spacer, a first linker, a second linker, and a third linker may be a unit that can be cleaved by cathepsin (e.g., cathepsin B, C or D).

In one embodiment of the present invention, a pH-sensitive linker comprising a group that induces the hydrolysis of an acid-labile group such as hydrazone may be comprised in the linker.

In one embodiment of the present invention, a glutathione-sensitive linker, such as a linker comprising a disulfide bridge, may be comprised.

In the present invention, an antibody and a linker may be conjugated via a thiol of the antibody, particularly, a thiol of cysteine. In addition, in the present invention, an antibody and a linker may be conjugated via an amine of the antibody, particularly, an amine of lysine.

In the present invention, when an antibody is conjugated with a linker unit, they may be conjugated by a structure comprising a spacer.

In one embodiment of the present invention, the conjugate may comprise a structure that is conjugated by click chemistry. Specifically, the conjugate may comprise a structure that is conjugated through an oxime bond mediated by carbonyl-aminooxy or azide-alkyne-based click chemistry, or a structure conjugated by the previous known method (Bioorg Med Chem. 2020 Dec 15; 28(24), Methods Mol Biol, 2020; 2078: 83-97 or the like).

In one embodiment of the present invention, the spacer indicates a direct bond or a chemical structure having 1 to 200 atoms.

In one embodiment of the present invention, the spacer is one or more selected from the group consisting of a first spacer, a second spacer, a third spacer, and a fourth spacer.

In one embodiment of the present invention, the spacer is two or more selected from the group consisting of a first spacer, a second spacer, a third spacer, and a fourth spacer.

In one embodiment of the present invention, the spacer is a combination of two or more selected from the group consisting of a first spacer, a second spacer, a third spacer, and a fourth spacer.

In one embodiment of the present invention, the spacer is a combination of a first spacer, a second spacer, a third spacer, and a fourth spacer.

In one embodiment of the present invention, the first spacer, the second spacer, the third spacer, or the fourth spacer is each independently selected from the group consisting of a direct bond, substituted or unsubstituted C₁₋₅₀ alkylene, substituted or unsubstituted C₁₋₅₀ heteroalkylene, substituted or unsubstituted C₂₋₅₀ alkenylene, substituted or unsubstituted C₃₋₅₀ cycloalkylene, substituted or unsubstituted C₆₋₅₀ arylene, and substituted or unsubstituted C₆₋₅₀ heteroarylene.

In one embodiment of the present invention, one or more of the first spacer, the second spacer, the third spacer, and the fourth spacer are unsubstituted C₁₋₁₀ heteroalkylene.

In one specific embodiment of the present invention, one or more of the first spacer, the second spacer, the third spacer, and the fourth spacer are unsubstituted C₁₋₁₀ heteroalkylene, and comprise an NH binding site.

In one embodiment of the present invention, one or more of the first spacer, the second spacer, the third spacer, and the fourth spacer are substituted C₁₋₂₀ heteroalkylene,
wherein when a group is substituted, the group may be substituted with one selected from the group consisting of halogen, =O, OH, NH₂, SH, NO₂, N₃, CN, OR₁, SR₁, OC(O)R₁, OC(O)NHR₁, OC(O)OR₁, CONHR₁, CON(R₁)₂, NHC(O)R₁, C(O)R₁, NHR₁, N(R₁)₂, C(O)R₁, OS(O)₂R₁, -OP(O)(OR₁)(OR₁), OP(O)(NHR₁)(NHR₁), and C₁₋₃ alkyl,
wherein R₁ is selected from the group consisting of H, OH, C₁₋₅ alkyl, C₁₋₅ heteroalkyl, C₃₋₈ aryl, and C₃₋₈ heteroaryl.

In one embodiment of the present invention, one or more of the first spacer, the second spacer, the third spacer, and the fourth spacer are substituted C₆₋₅₀ heteroarylene, wherein when a group is substituted, the group is substituted with one selected from the group consisting of halogen, =O, OH, NH₂, SH, NO₂, N₃, CN, OR₁, SR₁, OC(O)R₁, OC(O)NHR₁, OC(O)OR₁, CONHR₁, CON(R₁)₂, NHC(O)R₁, C(O)R₁, NHR₁, N(R₁)₂, C(O)R₁, OS(O)₂R₁, - OP(O)(OR₁)(OR₁), OP(O)(NHR₁)(NHR₁), and C₁₋₃ alkylene,
wherein R₁ is selected from the group consisting of H, OH, C₁₋₅ alkyl, C₁₋₅ heteroalkyl, C₃₋₈ aryl, and C₃₋₈ heteroaryl.

In one exemplary embodiment of the present invention, R₁ is H, OH, or C₁₋₃ alkyl.

In one embodiment of the present invention, one or more of the first spacer, the second spacer, the third spacer, and the fourth spacer are C₆₋₅₀ heteroarylene substituted with =O.

In one embodiment of the present invention, one or more of the first spacer, the second spacer, the third spacer, and the fourth spacer are direct bonds.

In one embodiment of the present invention, one or more of the first spacer, the second spacer, the third spacer, and the fourth spacer comprise terminal C(O).

In one embodiment of the present invention, one or more of the first spacer, the second spacer, the third spacer, and the fourth spacer comprise terminal NH.

In one embodiment of the present invention, one or more of the first spacer, the second spacer, the third spacer, and the fourth spacer comprise terminal C(O)NH.

In one embodiment of the present invention, one or more of the first spacer, the second spacer, the third spacer, and the fourth spacer are selected from the group consisting of - C(O)(CH₂)ₐ-, -NH(CH₂)ₐ-, -C(O)(CH₂)ₐC(O)-, -C(O)(CH₂)ₐNH-, -NH(CH₂)ₐNH-, - NHC(O)(CH₂)ₐC(O)-, -NHC(O)(CH₂)ₐNH-, -NHC(O)(CH₂)ₐC(O)NH-, and - NHC(O)(CH₂)ₐO(CH₂)_{b}-,
wherein a and b are each independently an integer from 1 to 10.

In one exemplary embodiment of the present invention, a and b are each independently an integer from 1 to 7.

In one embodiment of the present invention, one or more of the first spacer, the second spacer, the third spacer, and the fourth spacer comprise

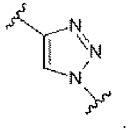

In addition, in one embodiment of the present invention, one or more of the first spacer, the second spacer, the third spacer, and the fourth spacer are 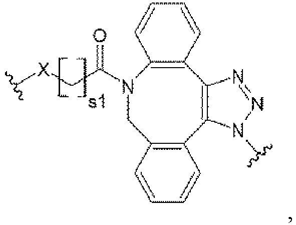 wherein X is substituted or unsubstituted C₁₋₁₀ alkyl or substituted or unsubstituted C₁₋₁₀ heteroalkyl, and when substituted, is substituted with one or more selected from the group consisting of halogen, =O, OH, NH₂, and SH, and s1 is an integer from 1 to 10.

In one embodiment of the present invention, one or more of the first spacer, the second spacer, the third spacer, and the fourth spacer are 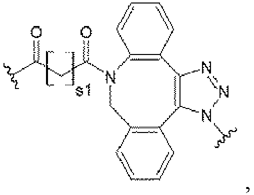 and s1 is an integer from 1 to 10.

In one embodiment of the present invention, one or more of the first spacer, the second spacer, the third spacer, and the fourth spacer are

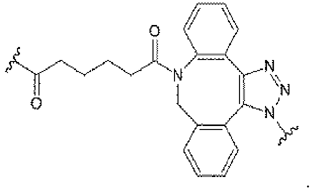

In one embodiment of the present invention, the spacer is 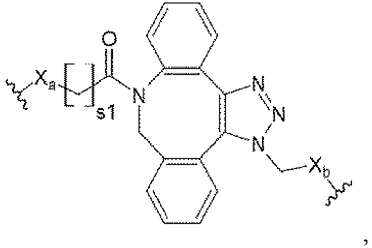 wherein Xₐ and X_{b} are each independently substituted or unsubstituted C₁₋₁₀ alkyl or substituted or unsubstituted C₁₋₁₀ heteroalkyl, and when a group is substituted, the group is substituted with one or more selected from the group consisting of halo, =O, OH, NH₂, and SH, and s1 is an integer from 1 to 10.

In one embodiment of the present invention, Xₐ and X_{b} are each independently selected from the group consisting of -C(O)(CH₂)ₐ-, -NH(CH₂)ₐ-, -C(O)(CH₂)ₐC(O)-, -C(O)(CH₂)ₐNH-, - NH(CH₂)ₐNH-, -NHC(O)(CH₂)ₐC(O)-, -NHC(O)(CH₂)ₐNH-, -NHC(O)(CH₂)ₐC(O)NH-, and - NHC(O)(CH₂)ₐO(CH₂)_{b}-,
wherein a and b are each independently an integer from 1 to 10.

In one embodiment of the present invention, Xₐ is -C(O)-.

In one embodiment of the present invention, X_{b} is -CH₂C(O)- or -(CH₂)₂OCH₂C(O)-NH-.

In one embodiment of the present invention, the spacer is -NH-(CH₂)ₐ-NH-, and s is an integer from 1 to 5.

In one embodiment of the present invention, Sp₁ is 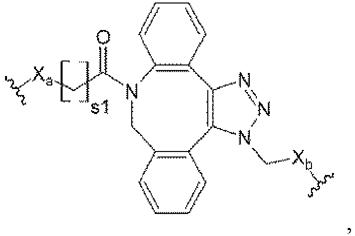 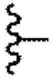 of Xₐ is bonded to L₁, and 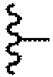 of X_{b} is bonded to Ab, and Xₐ, X_{b}, and s1 are defined as above.

In one embodiment of the present invention, L₁ and L₂ are each independently selected from the group consisting of a direct bond, an amino acid, a non-natural amino acid, and a derivative thereof.

In one embodiment of the present invention, L₁ and L₂ are each independently selected from the group consisting of a direct bond, alanine, β-alanine, γ-aminobutyric acid, arginine, asparagine, aspartic acid, γ-carboxyglutamic acid, citrulline, cysteine, glutamic acid, glutamine, glycine, histidine, isoleucine, leucine, lysine, methionine, norleucine, norvaline, ornithine, phenylalanine, proline, serine, threonine, tryptophan, tyrosine, valine, and derivatives thereof.

In one embodiment of the present invention, L₁ and L₂ are each independently selected from the group consisting of a direct bond, arginine, asparagine, glutamic acid, glutamine, glycine, alanine, arginine, citrulline, isoleucine, leucine, lysine, phenylalanine, serine, tryptophane, valine, and derivatives thereof.

In one embodiment of the present invention, L₁ and L₂ are each independently selected from the group consisting of a direct bond, arginine, asparagine, glutamic acid, glutamine, glycine, alanine, citrulline, lysine, phenylalanine, serine, and valine.

In one embodiment of the present invention, L₁ and L₂ are each independently selected from the group consisting of a direct bond, arginine, asparagine, glutamine, citrulline, lysine, serine, and valine.

In one embodiment of the present invention, L₁ and L₂ are each lysine.

In one embodiment of the present invention, L₁ is 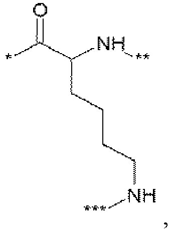 * and ** are sites that bind to another L₁, or bind to Sp₁ or Sp₄, and *** is a site that binds to L₂.

In one embodiment of the present invention, one or more of L₁ and L₂ can be cleaved by an enzyme.

In one embodiment of the present invention, one or more of L₁ and L₂ can be cleaved by a tumor-related protease.

In one embodiment of the present invention, one or more of L₁ and L₂ can be cleaved by glucuronidase. Specifically, one or more of L₁ and L₂ can be cleaved by β-glucuronidase.

In one exemplary embodiment of the present invention, one or more of L₁ and L₂ can be cleaved by cathepsin.

In one embodiment of the present invention, when L₂ in the conjugate is a direct bond, either -Sp₂-L₃-X₂ or -Sp₃-L₃'-X₃ is absent, and L₁ binds to either Sp₂-L₃-X₂ or -Sp₃-L₃'-X₃.

In one embodiment of the present invention, when all L₂ are direct bonds, the conjugate may be represented by Formula 2 below.

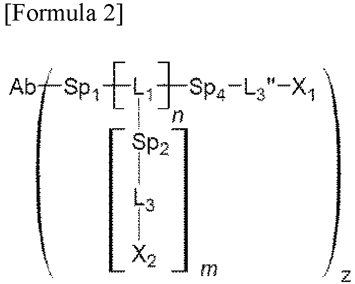

In the above formula,
Ab is a ligand,
Sp₁, Sp₂, and Sp₄ are each independently a spacer,
L₁ is a first linker unit,
L₃ and L₃" are each independently selected third linker units,
X₁, and X₂ are each independently selected active agent or polyethylene glycol unit,
wherein at least one of X₁ and X₂ is a polyethylene glycol unit,
z is an integer from 1 to 20,
n is an integer from 1 to 20, and
m is an integer from 1 to 20,
wherein when n and m are 2 or more, L₁, L₃, Sp₂, and X₂ are each independently selected.

In addition, here, each spacer, linker unit, active agent, and polyethylene glycol unit are defined as above.

In one embodiment of the present invention, one or more of L₃, L₃', and L₃" are direct bonds.

In one embodiment of the present invention, one or more of L₃, L₃', and L₃" are represented by Formula A.

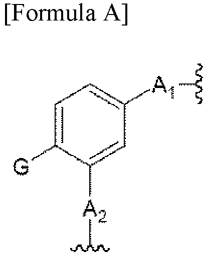

In the above formula,
G is glucuronic acid or a derivative thereof,
A₁ and A₂ are each independently selected from a group consisting of a direct bond, substituted or unsubstituted C₁₋₅₀ alkylene, substituted or unsubstituted C₁₋₅₀ heteroalkylene, substituted or unsubstituted C₂₋₅₀ alkenylene, substituted or unsubstituted C₃₋₅₀ cycloalkylene, substituted or unsubstituted C₆₋₅₀ arylene, and substituted or unsubstituted C₆₋₅₀ heteroarylene.

In one embodiment of the present invention, G is beta-glucuronic acid.

In one embodiment of the present invention, A₁ is bound to X₂ or X₃.

In one embodiment of the present invention, A₂ is bound to Sp₂, Sp₃, or Sp₄.

In one embodiment of the present invention, A₁ is selected from the group consisting of -(CH₂)ₐ₁OC(O)-(CH₂)_{b1}-, -(CH₂)ₐ₁C(O)-(CH₂)_{b1}-, -(CH₂)ₐ₁NH-(CH₂)_{b1}-, -(CH₂)ₐ₁C(O)NH-(CH₂)_{b1}, and -(CH₂)ₐ₁OC(O)NH-(CH₂)_{b1}-,
a1 and b 1 are each independently an integer from 0 to 10.

In one embodiment of the present invention, A₁ is -(CH₂)ₐ₁OC(O)-(CH₂)_{b1}-, and a1 and b1 are each independently an integer from 0 to 5.

In one embodiment of the present invention, A₁ is -CH₂OC(O)-.

In one embodiment of the present invention, A₂ is represented by Formula A-1.

[Formula A-1] -A₂₁-A₂₂-A₂₃-

A₂₁ and A₂₃ are each independently selected from the group consisting of -O-, -OCO-, - OCOO-, -OCONRₐ-, -NHRₐ-, -NRₐCO-, -NRₐCONR_{b}-, -NRₐCOO-, -NRₐCOR_{b}-, -NRₐCO-R_{b}-CO-, -NRₐCO-R_{b}-NH-, and -S(O)-,
wherein Rₐ and R_{b} are each independently H or C₁₋₅ alkylene, and
A₂₂ is C₁₋₁₀ alkylene or C₂₋₁₀ alkenylene.

In one embodiment of the present invention, A₂ is -NHC(O)-(CH₂)₂-NH-.

In the present invention, in the linker expressed as a combination of some of Sp₁, Sp₂, Sp₃, Sp₄, L₁, L₂, L₃, L₃', L₃", X₁, X₂, and X₃, one or more of X₁, X₂, and X₃ are polyethylene glycol units.

In the present invention, the polyethylene glycol units of the conjugate are bonded in a parallel form in an antibody-linker-drug as expressed in the combination form. In addition, in the present invention, two or more polyethylene glycol units may be included, and here, it does not mean that all the polyethylene glycol units are bonded in a parallel form. However, the polyethylene glycol units of the present invention may be bonded in a parallel form.

In one embodiment of the present invention, at least two or more of X₁, X₂, and X₃ are polyethylene glycol repeating units.

In one embodiment of the present invention, the polyethylene glycol unit comprises - (CH₂CH₂O)- as a repeating unit.

In one embodiment of the present invention, the polyethylene glycol unit comprises - (CH₂CH₂O)ₓ- as a repeating unit, and x is an integer from 4 to 40.

In one embodiment of the present invention, the polyethylene glycol unit comprises a - C(O)- binding site.

In one embodiment of the present invention, the polyethylene glycol unit comprises a terminal part of C₁₋₅ alkyl.

In one embodiment of the present invention, the polyethylene glycol unit may be represented by the following formula.

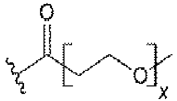

In the above formula, x is an integer from 4 to 40.

In one embodiment of the present invention, m is an integer of n or less, and m repeating units are each independently selected. In the present invention, the structural unit of m is bonded to the structural unit of n, and the structural unit of m corresponds to an integer less than or equal to the structural unit of n.

In one embodiment of the present invention, both n and m are 1.

In one embodiment of the present invention, both n and m are integers of 2 or more, and in this case, each structural unit may be selected independently from other structural units. For example, when n is 3, three L₁ are included, and the three L₁ may each be independently selected from the group consisting of an amino acid, a non-natural amino acid, and derivatives thereof to represent each of L₁. In addition, when m is 3, L₂, Sp₂, Sp₃, L₃', L₃", X₂, and X₃ represent a each independently selected unit, L₂ may each be independently selected from the group consisting of an amino acid, a non-natural amino acid, and derivatives thereof to represent three different L₁, and three X₂ and X₃ may each be independently an active agent or a polyethylene glycol unit.

In one embodiment of the present invention, n is an integer from 2 to 6.

In one embodiment of the present invention, n is an integer from 2 to 4.

In one embodiment of the present invention, m is an integer from 2 to 6.

In one embodiment of the present invention, m is an integer from 2 to 4.

In one embodiment of the present invention, at least one of X₁, X₂, and X₃ is an active agent.

In the present invention, as L₁ or L₂ increases, the polyethylene glycol units may increase. More specifically, when L₁ or L₂ increases by 1, the polyethylene glycol unit may increase by 1 or 2.

In one specific embodiment of the present invention, the conjugate may be a conjugate represented by Formula 1-2 below.

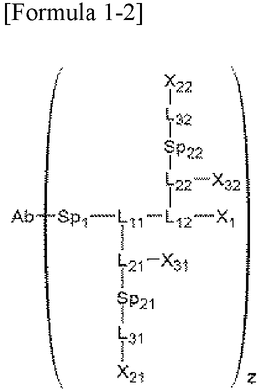

In the above formula, Ab, Sp₁, X₁ and z are the same as defined in Formula 1,
L₁₁, L₁₂ are the same as defined for L₁ in Formula 1,
L₂₁, L₂₂ are the same as defined for L₂ in Formula 1,
L₃₁, L₃₂ are the same as defined for L₃ in Formula 1,
Sp₂₁, Sp₂₂ are the same as defined for Sp₂ in Formula 1,
Sp₃₁, Sp₃₂ are the same as defined for Sp₃ in Formula 1,
X₂₁, X₂₂ are the same as defined for X₂ in Formula 1,
X₃₁, X₃₂ are the same as defined for X₃ in Formula 1.

In addition, in one exemplary embodiment of the present invention, the conjugate may be a conjugate represented by Formula 1-3 below.

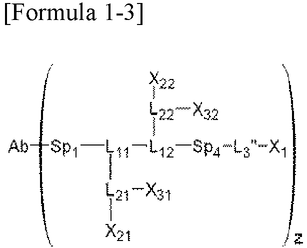

In the above formula, Ab, Sp₁ and Sp₄, L₃", X₁, and z are the same as defined in Formula 1,
L₁₁, L₁₂ are the same as defined for L₁ in Formula 1,
L₂₁, L₂₂ are the same as defined for L₂ in Formula 1,
X₂₁, X₂₂ are the same as defined for X₂ in Formula 1,
X₃₁, X₃₂ are the same as defined for X₃ in Formula 1.

In addition, in one specific embodiment of the present invention, the conjugate may be a conjugate represented by Formula 1-4 below.

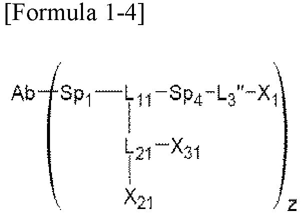

In the above formula, Ab, Sp₁, Sp₄, L₃", X₁, and z are the same as defined in Formula 1,
L₁₁ is the same as defined for L₁ in Formula 1,
L₂₁ is the same as defined for L₂ in Formula 1,
X₂₁ is the same as defined for X₂ in Formula 1, and
X₃₁ is the same as defined for X₃ in Formula 1.

In one specific embodiment of the present invention, the conjugate may be a conjugate represented by Formula 1-5 below.

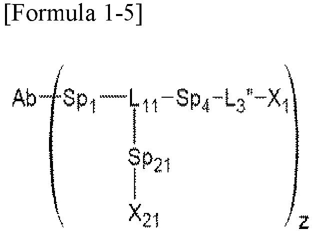

In the above formula, Ab, Sp₁, Sp₄, L₃", X₁, and z are the same as defined in Formula 1,
L₁₁ is the same as defined for L₁ in Formula 1,
Sp₂₁ is the same as defined for Sp₂ in Formula 1, and
X₂₁ is the same as defined for X₂ in Formula 1.

In one specific embodiment of the present invention, the conjugate may be a conjugate represented by Formula 1-6.

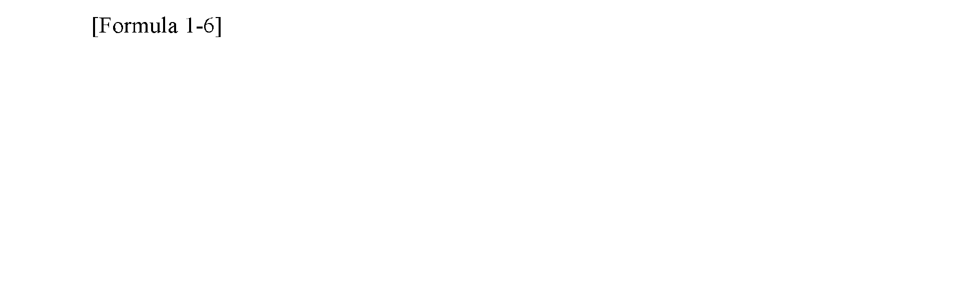

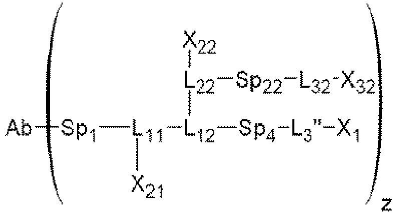

In the above formula, Ab, Sp₁, Sp₄, L₃", X₁, and z are the same as defined in Formula 1,
L₁₁, L₁₂ are the same as defined for L₁ in Formula 1,
L₂₂ is the same as defined for L₂ in Formula 1,
L₃₂ is the same as defined for L₃ in Formula 1,
Sp₂₂ is the same as defined for Sp₂ in Formula 1,
X₂₁, X₂₂ are the same as defined for X₂ in Formula 1, and
X₃₂ is the same as defined for X₃ in Formula 1.

In addition, the present invention relates to a linker unit represented by Formula 1a below.

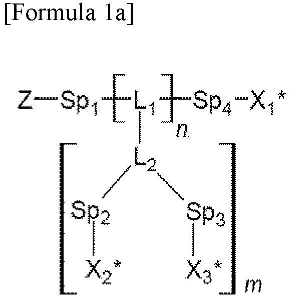

In the above formula,
Sp₁, Sp₂, Sp₃, and Sp₄ are each independently a spacer,
L₁ is a first linker unit,
L₂ is a second linker unit,
one or more of X₁*, X₂*, and X₃* are polyethylene glycol unit,
one or more of X₁*, X₂*, and X₃* are active agent-binding moiety if are not a polyethylene glycol unit,
n is an integer from 1 to 20,
m is an integer from 1 to 20,
wherein, when n and m are 2 or more, L₁, L₂, L₃, L₃', Sp₂, Sp₃, X₂*, and X₃* are each independently selected, and
Z is a ligand-binding moiety.

In the present invention, L₁, L₂, Sp₁, Sp₂, Sp₃, and Sp₄ of Formula 1a are the same as defined in Formula 1.

In addition, in the present invention, when X₁*, X₂*, and X₃* of Formula 1a are not active agent-binding moieties, they are defined the same as X₁, X₂ and X₃ of Formula 1.

In one embodiment of the present invention, at least two of X₁*, X₂*, and X₃* are polyethylene glycol units.

In one embodiment of the present invention, Z comprises an Fc-binding protein.

In one embodiment of the present invention, Z is 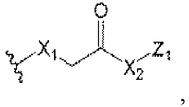
wherein X₁ is CR, NR, S, or O,
wherein R is hydrogen, C₁₋₃ alkyl, C₁₋₃ heteroalkyl, or C₁₋₃ alkoxy,
X₂ is NH, and Z₁ is an Fc-binding protein.

In one specific embodiment of the present invention, Z₁ is Ac-PEG8-DCAWH-(Dap)-GELVWCT-NH- (Ac: acetylation / Dap: di-amino-propionic acid).

In one specific embodiment of the present invention, Z₁ is Ac-PEG8-DCAWH-(Dap)-GELVWCT-NH- and comprises a Cys-Cys disulfide bond (disulfide oxidation).

In one embodiment of the present invention, the active agent-binding moiety is absent, or is selected from the group consisting of -(CH₂)_{z1}OC(O)-(CH₂)_{z2}-R_{z}, -(CH₂)_{z1}C(O)-(CH₂)_{z2}-R_{z}, -(CH₂)_{z1}NH-(CH₂)_{z2}-R_{z}, -(CH₂)_{z1}C(O)NH-(CH₂)_{z2}-R_{z}, -(CH₂)_{z1}NHC(O)-(CH₂)_{z2}-R_{z}, and - (CH₂)_{z1}OC(O)NH-(CH₂)_{z2}-R_{z},
wherein R_{z} is COOH, NH₂, C₂₋₅ alkynyl, C₅₋₂₀ cycloalkenyl, C₅₋₂₀ cycloalkynyl, C₅₋₂₀ heterocycloalkynyl, or N₃, and
z1 and z2 are each independently an integer from 0 to 10.

In one embodiment of the present invention, the active agent-binding moiety is 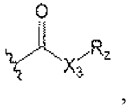
wherein X₃ is selected from the group consisting of -(CH₂)_{z1}OC(O)-(CH₂)_{z2}-R_{z}, - (CH₂)_{z1}C(O)-(CH₂)_{z2}-R_{z}, -(CH₂)_{z1}NH-(CH₂)_{z2}-R_{z}, -(CH₂)_{z1}C(O)NH-(CH₂)_{z2}-R_{z}, - (CH₂)_{z1}NHC(O)-(CH₂)_{z2}-R_{z}, and -(CH₂)_{z1}OC(O)NH-(CH₂)_{z2}-R_{z},
wherein R_{z} is COOH, NH₂, C₂₋₅ alkynyl, C₅₋₂₀ cycloalkynyl, C₅₋₂₀ heterocycloalkynyl, or N₃.

In one embodiment of the present invention, the active agent-binding moiety is -C(O)-CH₂-N₃.

In one exemplary embodiment of the present invention, the linker unit may be a linker unit represented by Formula 1-2a below.

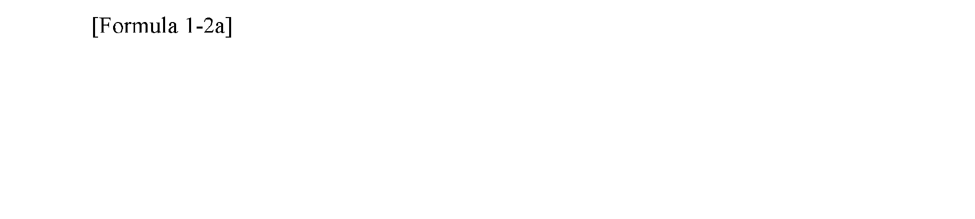

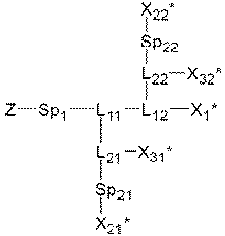

In the above formula, Z and Sp₁ are the same as Formula 1a,
L₁₁, L₁₂ are the same as L₁ of Formula 1a,
L₂₁, L₂₂ are the same as L₂ of Formula 1a,
Sp₂₁, Sp₂₂ are the same as Sp₂ of Formula 1a,
X₁*, X₂₁*, X₂₂*, X₃₁*, and X₃₂* are each independently a polyethylene glycol unit or an active agent-binding moiety.

In one specific embodiment of the present invention, the linker unit may be a linker unit represented by Formula 1-3a.

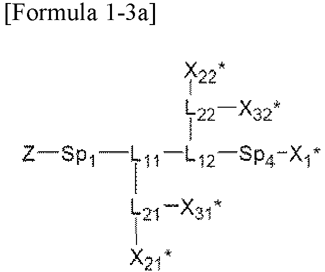

In the above formula, Z, Sp₁, and Sp₄ are the same as defined in Formula 1a,
L₁₁, L₁₂ are the same as L₁ of Formula 1a,
L₂₁, L₂₂ are the same as L₂ of Formula 1a,
X₁*, X₂₁*, X₂₂*, X₃₁*, and X₃₂* are each independently a polyethylene glycol unit or an active agent-binding moiety.

In one specific embodiment of the present invention, the linker unit may be a linker unit represented by Formula 1-4a below.

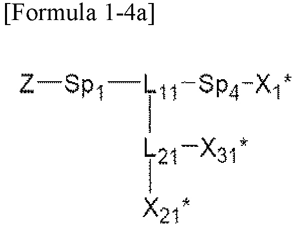

In the above formula, Z, Sp₁, and Sp₄ are the same as defined in Formula 1a,
L₁₁ is the same as L₁ of Formula 1a,
L₂₁ is the same as L₂ of Formula 1a,
X₁*, X₂₁*, and X₃₁* are each independently a polyethylene glycol unit or an active agent-binding moiety.

In one specific embodiment of the present invention, the linker unit may be a linker unit represented by Formula 1-5a below.

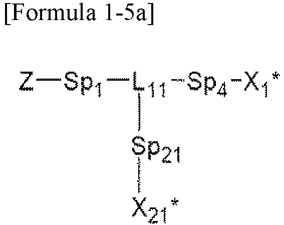

In the above formula, Z, Sp₁, and Sp₄ are the same as defined in Formula 1a,
L₁₁ is the same as L₁ of Formula 1a,
Sp₂₁ is the same as Sp₂ of Formula 1a,
X₁* and X₂₁* are each independently a polyethylene glycol unit or an active agent-binding moiety.

In one exemplary embodiment of the present invention, the linker unit may be a linker unit represented by Formula 1-6a below.

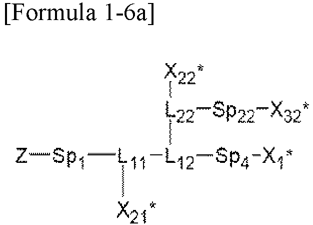

In the above formula, Z, Sp₁, and Sp₄ are the same as defined in Formula 1a,
L₁₁ and L₁₂ are the same as L₁ of Formula 1a,
L₂₂ is the same as L₂ of Formula 1a,
Sp₂₂ is the same as Sp₂ of Formula 1a,
X₁*, X₂₁*, X₂₂*, and X₃₂* are each independently a polyethylene glycol unit or an active agent-binding moiety.

In one embodiment of the present invention, the active agent is selected from the group consisting of a protein-targeting drug, a cell-targeting drug, a DNA-targeting drug, and an RNA-targeting drug.

In the present invention, each of the "protein-targeting drug," "cell-targeting drug," "DNA-targeting drug," and "RNA-targeting drug" refers to a drug that alters or kills the function of any protein, cell, DNA, or RNA in a harmful or harmless manner. More specifically, the protein-targeting drug, cell-targeting drug, DNA-targeting drug, and RNA-targeting drug may each be a toxic drug. The "toxic drug" refers to a drug that alters the function of any protein, cell, DNA, or RNA in a harmful manner. For example, it may alter cell growth, cell proliferation, protein synthesis, and DNA synthesis in a harmful manner.

In the present invention, non-limiting examples of drugs are as follows:
an antitubulin agent, an antibiotic, an antifolate, an alkylating agent, an anthracyclinebased activator, and a camptothecin-based activator
specifically, cyclophosphamide, mechlorethamine, chlorambucil, melphalan, carmustine (BCNU), lomustine (CCNU), ifofamine, procarbazine, dacarbazine, temezolomide, altretamine, cisplatin, carboplatin, taxol, taxotere, oxaliplatin, dolastatin 10, dolastatin 15, monomethyl auristatin E (MMAE), monomethyl auristatin F (MMAF), monomethyl auristatin D (MMAD), DM-1, DM-4, anthracycline, tubulysin, daunorubicin, doxorubicin, epirubicin, idarubicin, epirbucin, glarbuicin, daunorubicin, actinomycin, bleomycin, mitomycin, etoposide, valrubicin, mithramycin, mitoxantrone, paclitaxel, vincristine, vinblastine, vinorelbine, estramustine, nemorubicin, neocarzinostatin, docetaxel, puromycin, camptothecin, camtothecin, 10-hydroxyl camtothecin, cryptophycin, lurtotecan, gimatecan, topotecan, irinotecan, exatecan, orathecin, pirarbucin, silatecan, cositecan, gimatecan, belotecan, rubitecan, SN-38, maytansinoid, benzodiazepine, pyrrolobenzodiazepine, pyrrolobenzodiazepine dimer, calicheamicine, duocarmycin, methotrexate, amanitine, deoxycoformaycin, 6-mercaptopurine, 6-thioguanine, azathioprine, 2-chlorodeoxyadenosine, hydroxyurea, methotrexate, 5-fluorouracil, capecitabine, cytosine arabinoside, azacytidine, gemcitabine, fludarabine, asparaginase, FL118, afatinib

In one embodiment of the present invention, the active agent is auristatin.

In one exemplary embodiment of the present invention, the active agent is dolastatin, afatinib, duocarmycin, FL118, MMAD, MMAE, or MMAF.

In one embodiment of the present invention, the linker-active agent binding to the antibody is 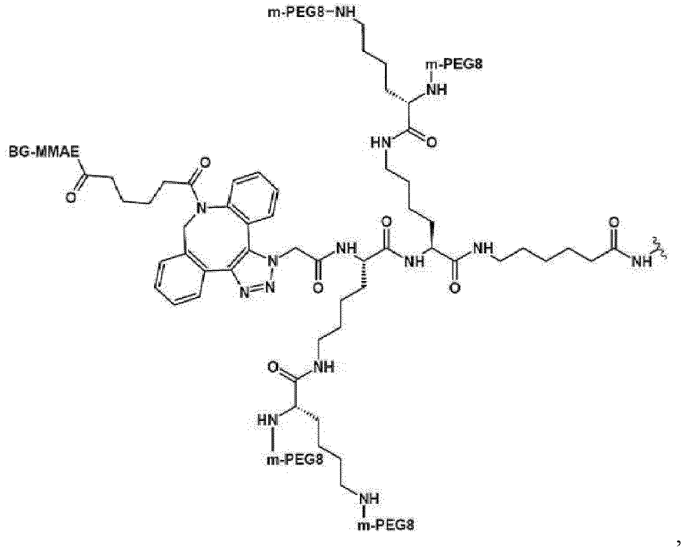 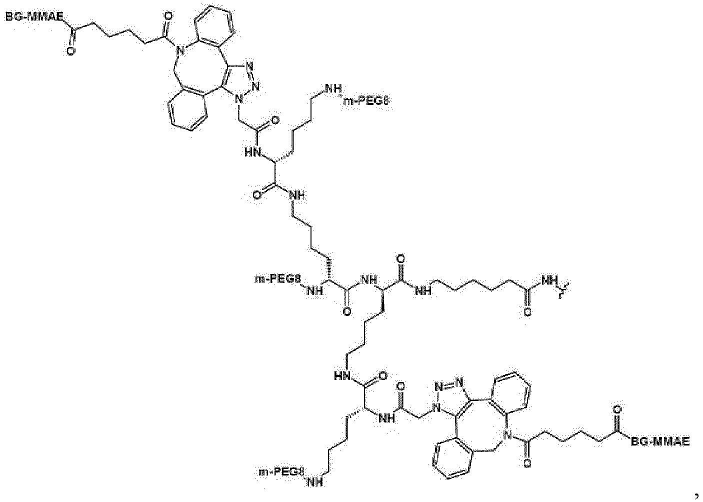 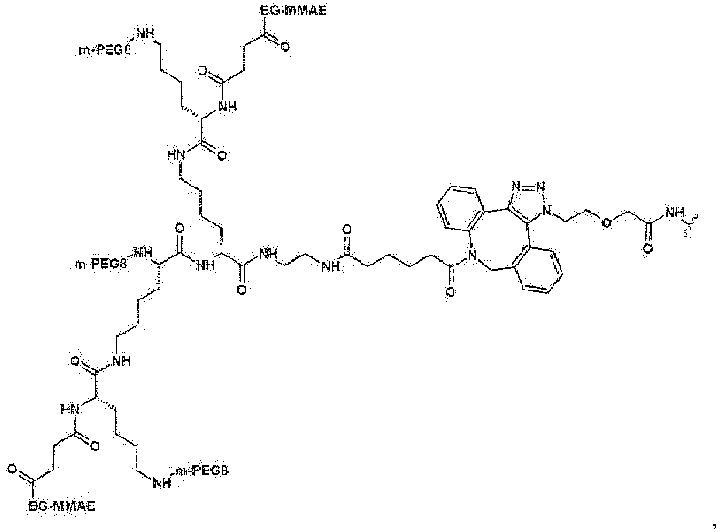 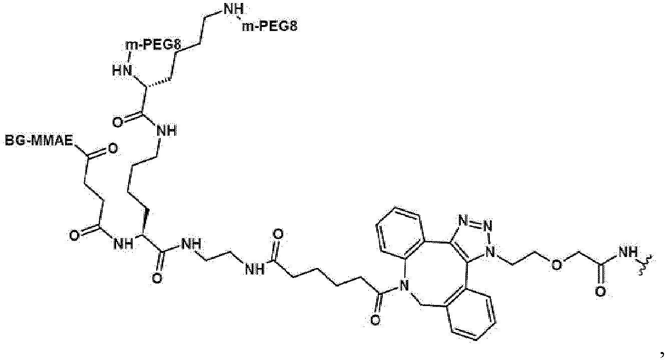 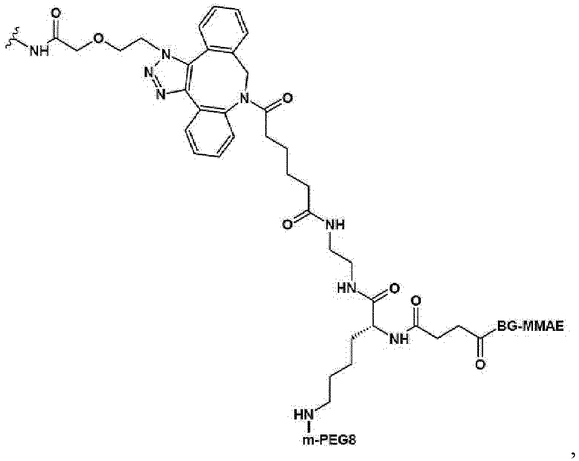 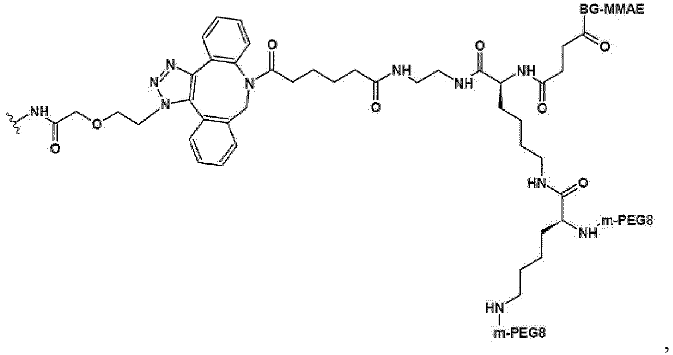 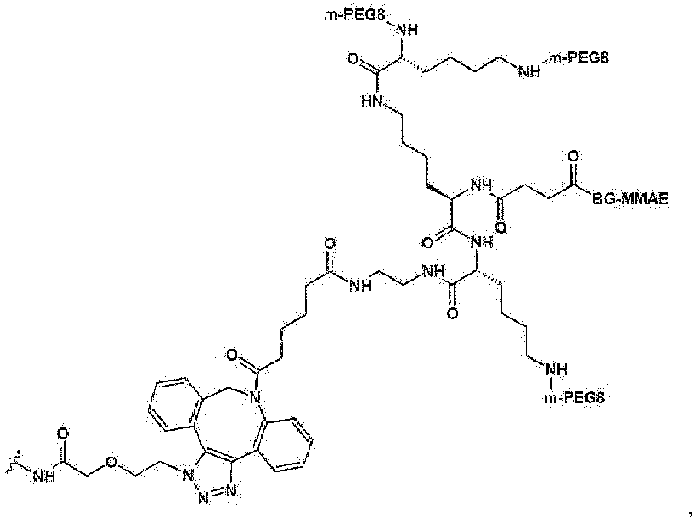 or 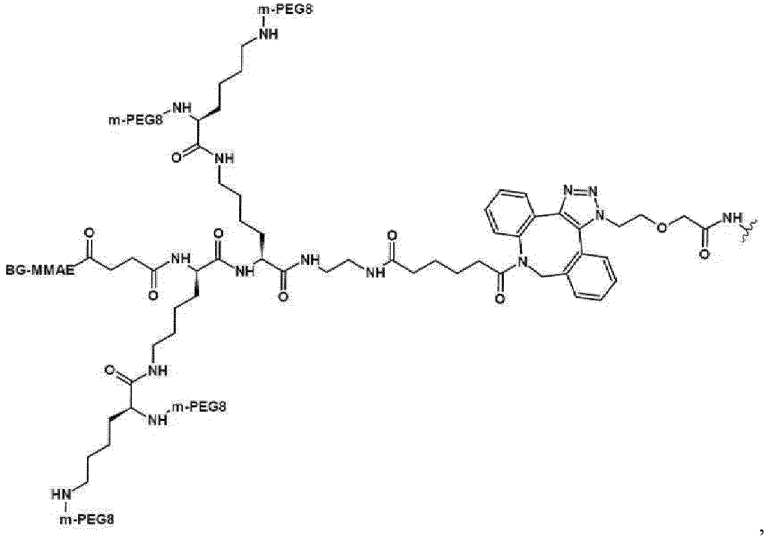 wherein
BG-MMAE is 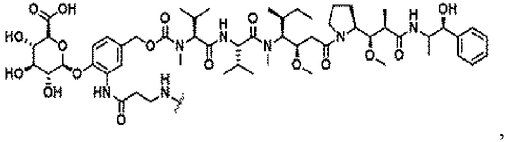 and m-PEG8 is

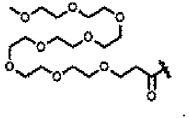

In addition, the present invention relates to a pharmaceutical composition comprising the conjugate.

In addition, the present invention relates to a pharmaceutical composition for preventing or treating a proliferative disease, comprising the conjugate.

In addition, the present invention relates to a method of treating a proliferative disease, which comprises administering a therapeutically effective amount of a composition comprising the conjugate to a subject in need of treatment of a proliferative disease.

In addition, the present invention relates to a use of the conjugate to prepare a medicament for treating a proliferative disease in a subject.

In one embodiment of the present invention, the proliferative disease is cancer.

Administration routes for the conjugate of the present invention may be, for example, oral or parenteral routes. Here, the parenteral routes mean the routes of administration in a broad sense, for example, intravenous, intra-arterial, intraperitoneal, intramuscular, subcutaneous, intranasal, sublingual, intrathecal, inhalation, ocular, rectal, vaginal, intra-cerebroventricular administration, and the like. When the composition is prepared, it is formulated using generally used diluents or excipients, such as a filler, an extender, a binder, a humectant, a disintegrant, and a surfactant.

Solid formulations for oral administration include tablets, pills, powders, granules, and capsules, and such solid preparations are prepared by mixing at least one excipient, such as starch, calcium carbonate, sucrose, lactose, or gelatin with the composition. Aside from simple excipients, lubricants such as magnesium stearate, talc, etc. are further used. Liquid formulations for oral administration include suspensions, liquids for internal use, emulsions, and syrups, and may further include various types of excipients, for example, a wetting agent, a sweetener, a fragrance and a preservative, other than a commonly used simple diluent such as water or liquid paraffin.

Formulations for parenteral administration include sterilized aqueous solutions, non-aqueous solvents, suspensions, emulsions, freeze-dried formulations, and suppositories. As a non-aqueous solvent or suspension, propylene glycol, polyethylene glycol, a vegetable oil such as olive oil, or an injectable ester such as ethyl oleate may be used. As a suppository base, Witepsol, Tween 61, cacao butter, laurin butter, glycerol, or gelatin may be used.

However, these are merely exemplary and are not limited thereto.

The composition of the present invention is administered at a pharmaceutically effective amount. The "pharmaceutically effective amount" used herein refers to an amount sufficient for treating a disease at a reasonable benefit/risk ratio applicable for medical treatment, and an effective dosage may be determined by parameters including a type of a patient's disease, severity, drug activity, sensitivity to a drug, administration time, an administration route and an excretion rate, the duration of treatment and drugs simultaneously used, and other parameters well known in the medical field. The composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without side effects, and such a dose may be easily determined by one of ordinary skill in the art.

### [Advantageous Effects]

A conjugate according to the present invention comprises a polyethylene glycol unit, so it can have excellent physical properties and prevent precipitation, resulting in increased drug delivery efficiency.

### [Description of Drawings]

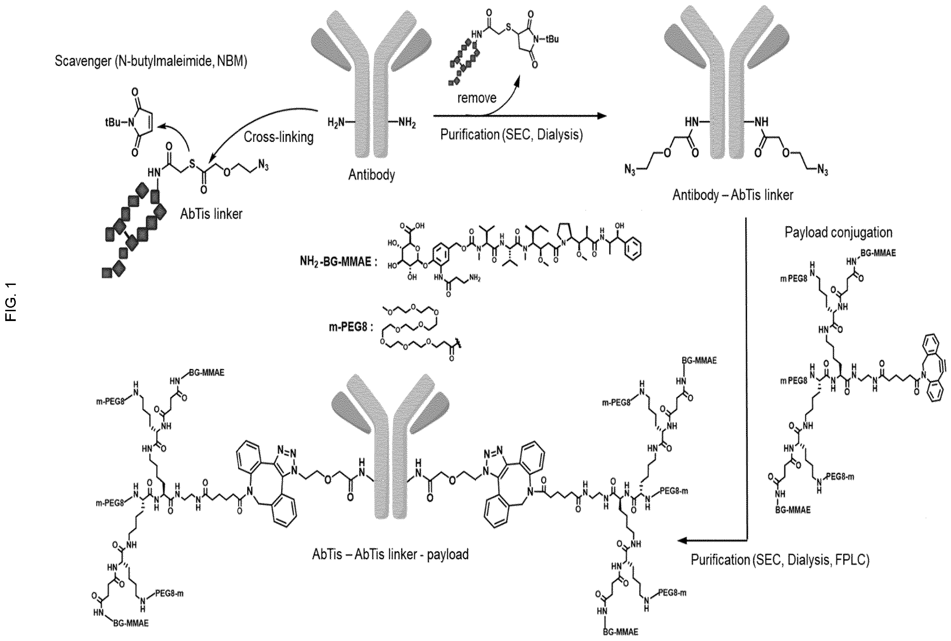

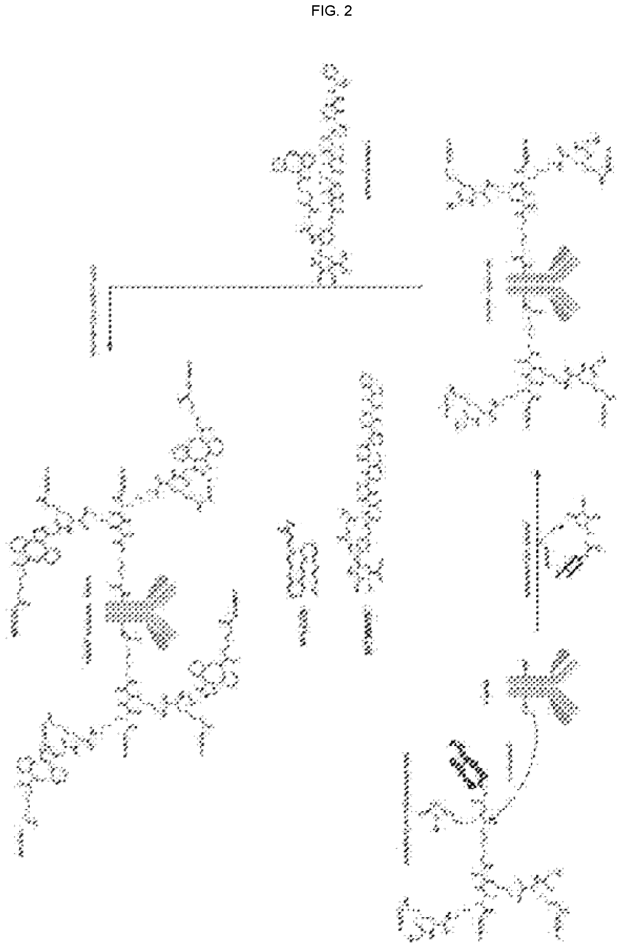

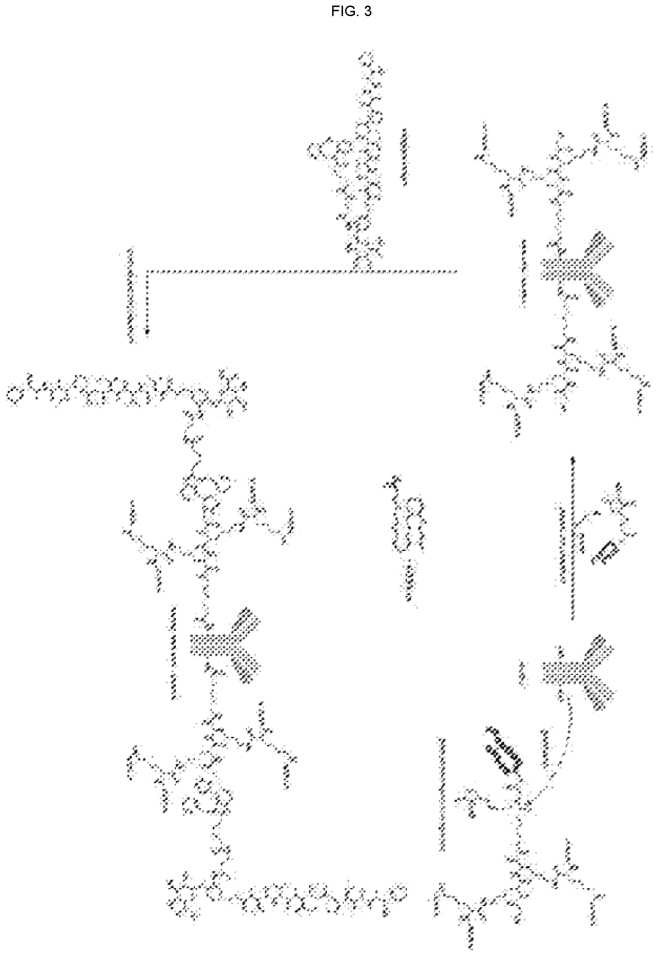

FIGS. 1 to 3 are diagrams illustrating the process of preparing a conjugate according to one embodiment of the present invention.

### [Modes of the Invention]

In the present invention, each term is defined as follows.

In the present invention, "independently" means that one selection does not depend on or affect another selection, so each can be selected.

In the present invention, "unit" refers to a unit that is segmented or can be segmented.

In the present invention, "analog" and "derivative" refer to a compound in which some parts are removed, modified, or added to the parent compound.

In the specification, "linker" refers to the entire chemical moiety that connects a molecule or atom to another molecule, for example, an antibody and an active agent. The linker may refer to a form in which multiple molecules or amino acids are bonded via a chemical functional group that can be covalently bonded.

In the specification, "cleavable" has the same chemical/biological meaning as known in the art, and includes cleavage under specific conditions, for example, acidic, reducing, enzymatic, etc. conditions.

In the specification, "unsubstituted or substituted" means a parent group that may be unsubstituted or may be substituted, "substituted" means a parent group having one or more substituents, and "substituent" refers to a chemical moiety that is covalently bound or fused to a parent group.

In the present invention, "alkyl" is a monovalent moiety obtained by removing a hydrogen atom from a carbon atom of an aliphatic or alicyclic, saturated, or unsaturated (unsaturated, completely unsaturated) hydrocarbon compound, and examples of saturated alkyls may include methyl, ethyl, propyl, butyl, pentyl, hexyl, and heptyl, examples of saturated linear alkyls may include methyl, ethyl, n-propyl, n-butyl, n-pentyl(amyl), n-hexyl, and n-heptyl, and examples of saturated branched alkyls may include isopropyl, isobutyl, sec-butyl, tert-butyl, isopentyl, and neopentyl.

In the present invention, "alkenyl" is alkyl having one or more carbon-carbon double bonds, and examples of unsaturated alkenyl groups may include ethenyl (vinyl, -CH=CH₂), 1-propenyl (-CH=CHCH₃), 2-propenyl, isopropenyl, butenyl, pentenyl, and hexenyl.

In the present invention, "alkynyl" is an alkyl group having one or more carbon-carbon triple bonds, and examples of unsaturated alkynyl groups may include ethynyl and 2-propynyl.

In the present invention, "alkylene" refers to a divalent moiety that is obtained by further removing one hydrogen atom from a carbon atom in the alkyl.

In the present invention, "alkenylene" refers to a divalent moiety obtained by further removing one hydrogen atom from a carbon atom in the alkenyl.

In the present invention, "alkynylene" refers to a divalent moiety obtained by further removing one hydrogen atom from a carbon atom in the alkynyl.

In the present invention, "halo" refers to fluorine, chlorine, bromine, or iodine.

In the present invention, "aryl" is a monovalent moiety obtained by removing a hydrogen atom from an aromatic ring atom of an aromatic compound. For example, "C₅₋₇ aryl" refers to a monovalent moiety that has 5 to 7 ring atoms and is obtained by removing a hydrogen atom from an aromatic ring atom of an aromatic compound, and "C₅₋₁₀ aryl" refers to a monovalent moiety that has 5 to 10 ring atoms and is obtained by removing a hydrogen atom from an aromatic ring atom of an aromatic compound. Here, the prefix (C₅₋₇, C₅₋₁₀ or the like) refers to the number of ring atoms or a range of the number of ring atoms regardless of whether it is a carbon atom or heteroatom. For example, "C₅₋₆ aryl" refers to an aryl group having 5 or 6 ring atoms. Here, the ring atoms may all be carbon atoms as in the "carboaryl group." Examples of the carboaryl groups include those derived from benzene, naphthalene, azulene, anthracene, phenanthrene, naphthacene, and pyrene, but the present invention is not limited thereto. Examples of aryl groups, comprising a fused ring, at least one of which is an aromatic ring, include groups derived from indane, indene, isoindene, tetralin, acenaphthene, fluorene, phenalene, acephenanthrene, and aceanthrene, but the present invention is not limited thereto. Alternatively, the ring atom may comprise one or more heteroatoms as in a "heteroaryl group."

In the present invention, "heteroaryl" refers to an aryl comprising one or more heteroatoms, and may include, for example, pyridine, pyrimidine, benzothiophene, furyl, dioxalanyl, pyrrolyl, oxazolyly, pyridyl, pyridazinyl, and pyrimidinyl, and more specifically, C₉ having two fused rings derived from benzofuran, isobenzofuran, indole, isoindole, indolizine, indoline, isoindoline, purine (adenine or guanine), benzimidazole, indazole, benzoxazole, benzisoxazole, benzodioxole, benzofuran, benzotriazole, benzothiofuran, benzothiazole, and benzothiadiazole; C₁₀ having two fused rings derived from chromene, isochromene, chromane, isochroman, benzodioxane, quinoline, isoquinoline, quinolizine, benzoxazine, benzodiazine, pyridopyridine, quinoxaline, quinazoline, cinnoline, phthalazine, naphthyridine, and pteridine; C₁₁ having two fused rings induced from benzodiazepine; C₁₃ having three fused rings derived from carbazole, dibenzofuran, dibenzothiophene, carboline, perimidine, and pyridoindole; and C₁₄ having three fused rings derived from acridine, xanthene, thioxanthene, oxanthrene, phenoxathiin, phenazine, phenoxazine, phenothiazine, thiantrene, phenanthridine, phenanthroline, and phenazine.

In the present invention, "cycloalkyl" is a cyclic alkyl group and refers to a monovalent moiety obtained by removing a hydrogen atom from an alicyclic ring atom of a cyclic hydrocarbon compound. Examples of cycloalkyl groups include the following compounds and derivatives thereof, but the present invention is not limited thereto. "Cycloalkyl" may include cyclopropane, cyclobutane, cyclopentane, cyclohexane, cycloheptane, methylcyclopropane, dimethylcyclopropane, methylcyclobutane, dimethyl cyclobutane, methyl cyclopentane, dimethyl cyclopentane, and methyl cycloheptane; unsaturated monocyclic hydrocarbon compounds such as cyclopropene, cyclobutene, cyclopentene, cyclohexene, methyl cyclopropene, dimethyl cyclopropene, methyl cyclobutene, dimethyl cyclobutene, methyl cyclopentene, dimethyl cyclopentene, and methyl cyclohexene; and saturated heterocyclic hydrocarbon compounds such as such as norcarane, norpinane, and norbornane.

In the present invention, "arylene" refers to a divalent moiety obtained by further removing one hydrogen atom from a carbon atom in the aryl.

In the present invention, "heteroarylene" refers to a divalent moiety obtained by further removing one hydrogen atom from a carbon atom in the heteroaryl.

In the present invention, the prefix (e.g., C₁₋₁₂, C₃₋₈, or the like) refers to the number of ring atoms or a range of the number of ring atoms regardless of whether it is a carbon atom or heteroatom. For example, the term "C₃₋₆ heterocyclyl" used herein refers to a heterocyclyl group having 3 to 6 ring atoms, and here, the type of ring atom is not limited thereto.

In the present invention, "solvate" refers to a molecular complex between the compound according to the present invention and solvent molecules, and examples of solvates comprise the compound according to the present invention combined with water, isopropanol, ethanol, methanol, dimethyl sulfoxide, ethyl acetate, acetic acid, ethanolamine, or a mixed solvent thereof, but the present invention is not limited thereto.

In the present invention, "pharmaceutically acceptable salt" refers to a salt form of the compound according to the present invention having a desired pharmacological activity. The salt forms include inorganic acids (hydrochloric acid, hydroboromic acid, sulfuric acid, nitric acid, and phosphoric acid), and organic acids (acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, and benzoic acid), but the present invention is not limited thereto.

In the present invention, "pharmaceutically effective amount" refers to an amount sufficient for preventing or treating a disease at a reasonable benefit/risk ratio applicable for medical prevention or treatment, and an effective dosage may be determined by parameters including the severity of a disease, drug activity, a patient's body weight, health, sex, and drug sensitivity, the administration time, route, and excretion rate of the composition of the present application used herein, the duration of treatment, and a drug that is combined or concurrently used with the composition of the present application used herein, and other parameters well known in the medical field.

The present invention may improve drug properties by arranging specific units in parallel in an antibody-drug conjugate.

The present invention may be designed to enable the efficient release of an active compound by binding to a specific amino acid position of an antibody Fc domain, and parts of a linker and an active agent may be controlled to bind to a specific amino acid position.

To control the orientation and binding positions of polyethylene glycol units, an appropriate linker or functional group may be introduced, and accordingly, the selectivity, pharmacokinetics, etc. of the antibody-drug conjugate may be different. Specifically, to control the parallel orientation and binding positions of polyethylene glycol units, a compound comprising an amino acid having an amine functional group or an amine functional group may be used. The polyethylene glycol units may be increased or decreased depending on the number of amino acids having an amine functional group. In addition, the polyethylene glycol unit may be increased or decreased depending on the position and structure of an amino acid having an amine functional group.

In the present invention, through parallel orientation of polyethylene glycol units, the hydrophobic portion of the drug may be shielded or masked, enabling the efficient release of the drug in target cells. Therefore, enhancement effects such as PK enhancement and improved *in vivo* efficacy are shown. The polyethylene glycol units may be used at a position or in a number suitable for shielding or masking of the hydrophobic portion of the drug, and this may vary depending on the type of drug.

In a specific embodiment, a thioester functional group may be introduced to enable binding to the Fc domain 248 of an IgG 1, 3 or 4 antibody. In addition, to increase reactivity, a compound comprising a maleimide functional group or a thiol-reactive additive compound may be used.

In a specific embodiment, to remove interfering factors such as hydrogen bonding that can occur when a cyclic peptide binds to an antibody, a C6 spacer (e.g., a C6 alkyl structure) may be introduced and bound to the lysine at amino acid position 248. In addition, after introducing a functional group by introducing the spacer at amino acid position 248, a drug may be introduced, and accordingly, a three-dimensional masking or shielding effect may be exhibited.

In a specific embodiment, the amino acid having an amine functional group may be lysine. However, this is only an example which can be implemented, and the present invention is not limited thereto.

Hereinafter, the present invention will be described in detail with reference to examples and experimental examples.

However, the following examples and experimental examples are merely illustrative, and the content of the present invention is not limited to the following examples and experimental examples.

### [Examples]

### <Example 1-1> Preparation of linker (PEG BRNAched payload linker)

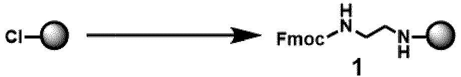

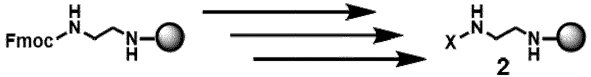

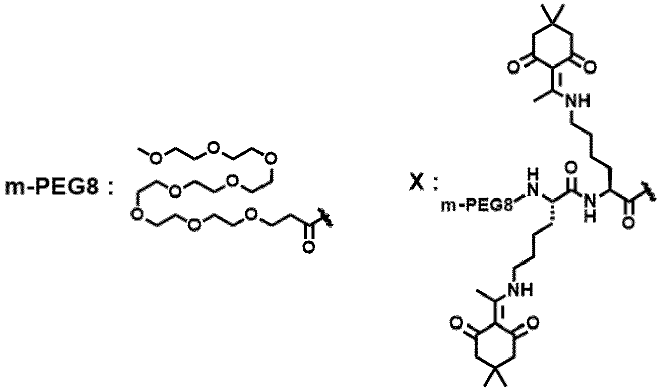

### Preparation of Compound 1

50 mL of dichloromethane was added to 2 g of 2-chlorotrityl chloride resin (1.4 mmol/g) and then stirred for 30 minutes. Subsequently, ethylene-di-amine (5.6 mmol, 2 eq) and N,N'-diisopropylethylamine (11.2 mmol, 4 eq) were mixed with 40 mL of dichloromethane, and then added to the resin. After the resulting mixture was stirred at room temperature for 2 hours, a reaction solution was removed. Afterward, 50 mL each of dichloromethane and dimethylformamide was added three times to wash the remaining resin, thereby preparing Compound 1.

### Preparation of Compound 2

N-alpha-Fmoc-N-epsilon-[1-(4,4-dimethyl-2,6-dioxycyclohex-1-ylidine)ethyl]-L-lysine (5.6 mmol, 2 eq), hydroxybenzotriazole (11.2 mmol, 4 eq), and N,N'-diisopropylcarbodiimide (5.6 mmol, 2 eq) were added to the resin containing Compound 1 and the resulting mixture was stirred for 2 hours at room temperature by using 50 mL of dimethylformamide as a solvent, and then the reaction solution was removed. Subsequently, this process was repeatedly performed in the same manner as described above by sequentially adding amino acid N-alpha-Fmoc-N-epsilon-[1-(4,4-dimethyl-2,6-dioxycyclohex-1-ylidine)ethyl]-L-lysine and 4,7,10,13,16,19,22,25-octaoxahexacosanoic acid. After the reaction of 4,7,10,13,16,19,22,25-octaoxahexacosanoic acid, 50 mL each of dichloromethane and dimethylformamide was added three times to wash the resin, thereby preparing Compound 2.

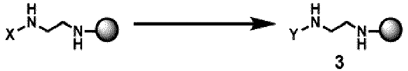

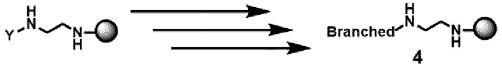

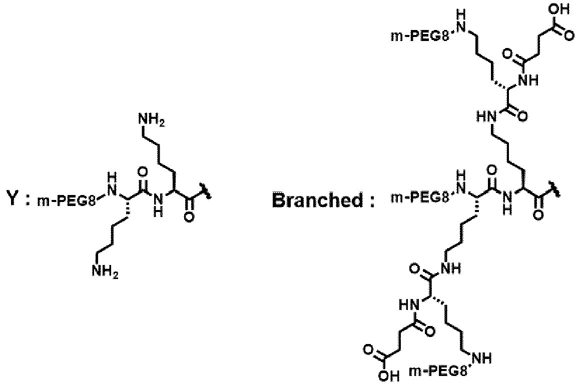

### Preparation of Compound 3

After 50 mL of 5% (volume ratio) hydrazine-containing dimethylformamide was added to the resin and stirred at room temperature for 10 minutes, the reaction solution was removed. Subsequently, 50 mL each of dichloromethane and dimethylformamide was added three times to wash the resin, thereby preparing Compound 3.

### Preparation of Compound 4

N-alpha-Fmoc-N-epsilon-[1-(4,4-dimethyl-2,6-dioxycyclohex-1-ylidine)ethyl]-L-lysine (11.2 mmol, 4 eq), hydroxybenzotriazole (22.4 mmol, 8 eq), and N,N'-diisopropylcarbodiimide (11.2 mmol, 4 eq) were added to the resin containing Compound 3 and the resulting mixture was stirred for 2 hours at room temperature by using 50 mL of dimethylformamide as a solvent, and then the reaction solution was removed. After the reaction, 40 mL of a 20% (volume ratio) piperidine-containing dimethylformamide solution was added to the resin and stirred for 10 minutes at room temperature, and then the solution was removed (repeated twice). Afterward, 50 mL each of dichloromethane and dimethylformamide was added three times to wash the resin. For the next reaction, succinic anhydride (16.8 mmol, 6 eq) was added, diisopropylethylamine (16.8 mmol, 6 eq) was added to the resin, and then 50 mL of dimethylformamide as a solvent was added and stirred for 1 hour at room temperature, followed by removing the reaction solution. After the reaction, 50 mL each of dichloromethane and dimethylformamide was added three times to wash. The following process was performed in the same manner as the process of preparing Compound 3. Subsequently, 2,5,8,11,14,17,20,23-octaoxahexacosane-26-oic-acid (28 mmol, 2 eq), hydroxybenzotriazole (56 mmol, 4 eq), and N,N'-diisopropylcarbodiimide (28 mmol, 2 eq) were sequentially added, 50 mL of dimethylformamide as a solvent was added to perform a reaction activation process for 10 minutes, and then the resulting product was added to the resin. After stirring for 2 hours at room temperature, the reaction solution was removed. After the reaction was completed, 50 mL each of dichloromethane and dimethylformamide was added three times to wash the resulting product, and then the resin was dried to prepare Compound 4.

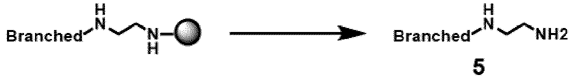

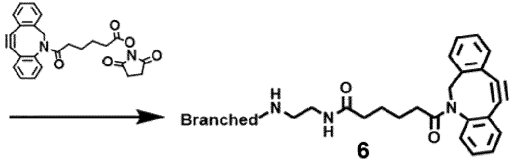

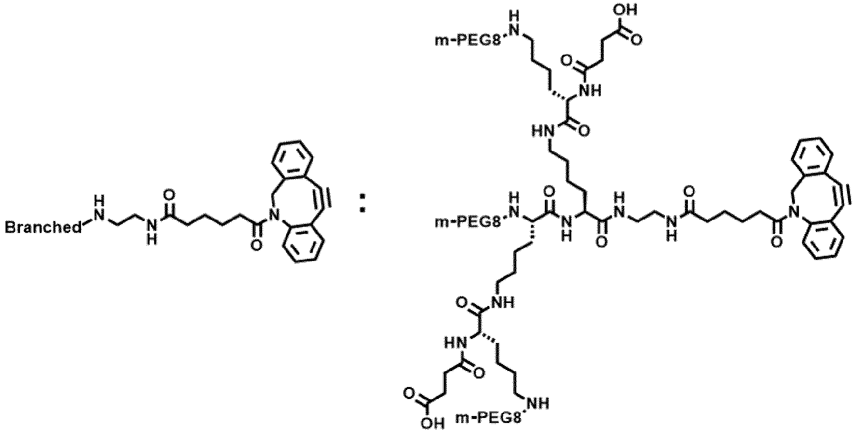

### Preparation of Compound 5

50 mL of trifluoroacetic acid (TFA), triisopropylsilane (TIS) and deionized water (DW) mixed in a volume ratio of 95 : 2.5 : 2.5 (TFA:TIS:DW) was added to the resin which had been processed until the preparation of Compound 4, and then stirred at room temperature for 2 hours. After stirring, the solution and the resin were separated, 500 mL of 0 °C diethyl ether was added to the separated solution and then mixed, and the resulting mixture was left at 0 °C for 2 or more hours. After filtering the precipitated product, 50 mL of 0 °C diethyl ether was added and filtered again (repeated twice). Afterward, the resulting product was dried to prepare Compound 5 (2.24 mmol, 80%).

### Preparation of Compound 6

After Compound 5 (1 g, 0.511 mmol) and (2,5-dioxo-1-pyrrolidinyl 11,12-didehydro-ε-oxodibenz[b,f]azocine-5(6H)-hexanoate (DBCO-C6-NHS; 0.613 mmol, 1.2 eq) were mixed in 10 mL of dimethylformamide, diisopropylethylamine (1.022 mmol, 2 eq) was added to the resulting mixture and stirred at room temperature for 1 hour. After the reaction was completed, trifluoroacetic acid was added until the pH of the solution reached 5.0. The reaction solution was purified by Prep-HPLC (C18), thereby obtaining Compound 6 (0.434 mmol, 85%).

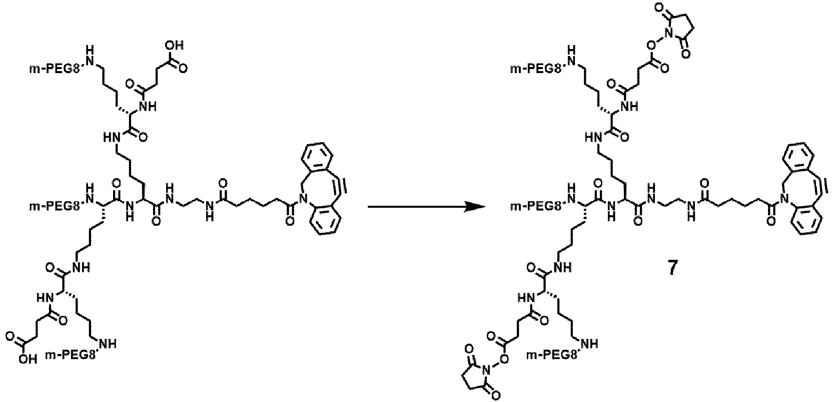

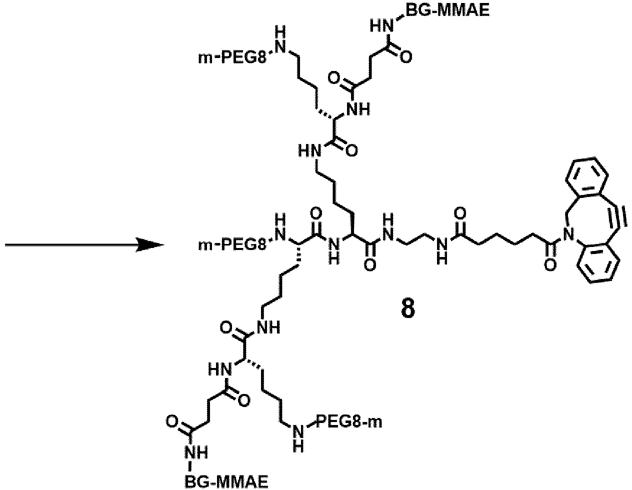

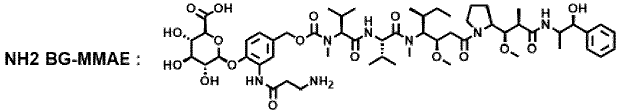

### Preparation of Compound 7

Compound 6 (0.434 mmol) and tetramethyl-O-(N-succinimidyl)uronium tetrafluoroborate (1.736 mmol, 4 eq) were mixed with 10 mL of dimethylformamide, diisopropylethylamine (1.736 mmol, 4 eq) was added, and the resulting mixture was stirred at room temperature for 0.5 to 2 hours. After the reaction was completed, trifluoroacetic acid was added until the pH of the solution reached 5.0. The reaction solution was purified by Prep-HPLC (C18) and freeze-dried, thereby obtaining Compound 7.

### Preparation of Compound 8

After mixing prepared H₂N-BG-MMAE (0.26 mmol, 1.2 eq) with the synthesized Compound 7 (0.217 mmol) in 5 mL of dimethylformamide, diisopropylethylamine (0.434 mmol, 2 eq) was added, and the resulting mixture was stirred at room temperature for 1 hour. After the reaction was completed, trifluoroacetic acid was added until the pH of the solution reached 5.0. The reaction solution was purified by Prep-HPLC (C18) and freeze-dried, thereby obtaining Compound 8. Compound 8 was dried, sealed, and then stored at -80 °C.

### <Example 1-2> Preparation of NH₂-beta glucuronide-monomethyl auristatin E (NH₂-BG-MMAE)

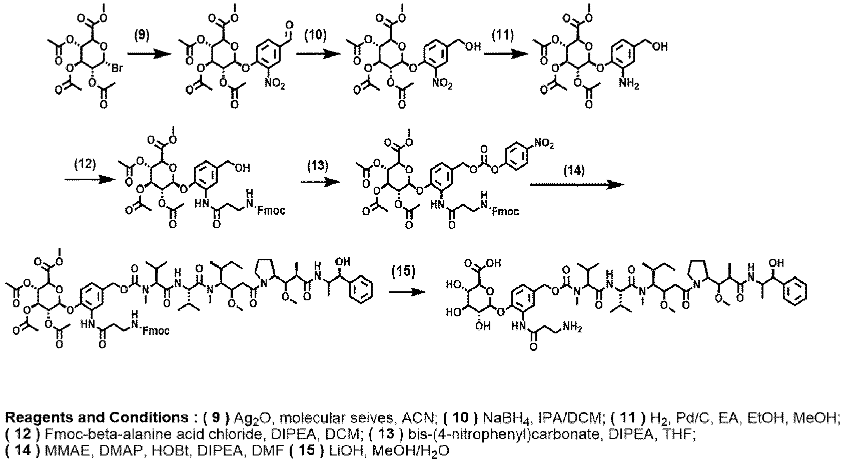

### Preparation of Compound 9

4-hydroxy-3-nitrobenzaldehyde (4 g, 29.6 mmol) was dissolved in 120 mL of acetonitrile, and then a molecular-sieve (10 g) was added. Afterward, acetobromo-α-D-glucuronic acid methyl ether (10.3 g, 32.6 mmol) and silver oxide (I) (7 g, 30.2 mmol) were added and stirred under an argon atmosphere for 18 hours. The reaction solution was filtered through Celite, concentrated under reduced pressure, and the organic layer was extracted with ethyl acetate and distilled water. The collected organic layer was dried over anhydrous magnesium sulfate. After filtration, concentration and purification by column chromatography were performed, thereby obtaining Compound 9 (12.6 g, 88%).

### Preparation of Compound 10

After dissolving Compound 9 (12.6 g, 26.0 mmol) in isopropyl alcohol (50.6 mL) and chloroform (185 mL), sodium borohydride (1.67 g, 44.1 mmol) was slowly added at 0 °C under a nitrogen atmosphere and then stirred for 2.5 hours. Deionized water was added to the reaction solution and stirred for 15 minutes. Ethyl acetate and deionized water were added to extract an organic layer. The collected organic layer was dried over anhydrous magnesium sulfate. After filtration, concentration and purification by column chromatography were performed, thereby obtaining Compound 10 (7.7 g, 61.1 %).

### Preparation of Compound 11

After dissolving Compound 10 (6.7 g, 14.2 mmol) in ethyl acetate (90 mL), ethanol (190 mL), and methanol (44 mL), palladium (0.33 g) was added and stirred in a hydrogen atmosphere for 3 hours. The reaction solution was filtered through Celite, concentrated under reduced pressure, and then purified by column chromatography, thereby obtaining Compound 11 (5.3 g, 76.9%).

### Preparation of Compound 12

Fluorenylmethyloxycarbonyl-beta-alanine (Fmoc-beta-alanine) (2.46 g, 7.9 mmol) was dissolved in dichloromethane (13 mL) and N,N-dimethylformamide (0.5 mL), oxalyl chloride (1.36 mL, 15.8 mmol) was slowly added under a nitrogen atmosphere, and the resulting mixture was stirred for 2 hours. After concentrating the reaction solution under reduced pressure, the resulting concentrate was slowly added to Compound 3 (3g, 6.59mmol), which had been dissolved in dichloromethane (33 mL), at 0 °C. Diisopropylethylamine (2.68 mL, 7.9 mmol) was slowly added to the dissolved compound at 0 °C. The reaction solution was concentrated and then purified by column chromatography, thereby obtaining Compound 12 (3.9 g, 79.1%).

### Preparation of Compound 13

After dissolving Compound 12 (2.5 g, 3.34 mmol) in dichloromethane (30 mL), bis(4-nitrophenyl)carbonate (1.73 g, 5.7 mmol) and N,N-diisopropylethylamine (0.99 mL, 5.68 mmol) were added and stirred for 2 hours. The reaction solution was concentrated under reduced pressure and purified by column chromatography, thereby obtaining Compound 13 (2.3 g, 75.4%).

### Preparation of Compound 14

After dissolving Compound 13 (1 g, 1.09 mmol) in N,N-diisopropylethylamine (10.8 mL), monomethyl auristatin E (MMAE, 0.864 g, 1.2 mmol), N-hydroxybenzotriazole hydrate (HOBt.H₂O, 0.44 g, 3.27 mmol), and N,N-diisopropylethylamine (0.28 mL, 1.64 mmol) were added and the resulting mixture was stirred at room temperature for 18 hours. The reaction solution was concentrated under reduced pressure and purified by column chromatography, thereby obtaining Compound 14 (1.45 g, 89%).

### Preparation of Compound 15

After dissolving Compound 14 (1.4 g, 9.37 mmol) in methanol (15.2 mL), a 0.4 Nlithium hydroxide solution (1.5 mL) was added to resulting mixture and then stirred for 6 hours. The reaction solution was purified by column chromatography, thereby obtaining Compound 15 (0.62 g, 59%).

### <Example 2-1> Preparation of linker (C6PEG3 linker)

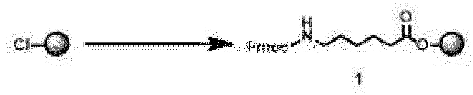

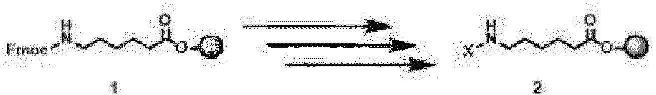

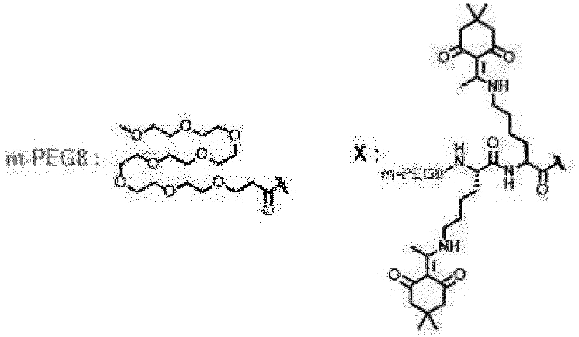

### Preparation of Compound 1

50 mL of dichloromethane was added to 5 g of 2-chlorotrityl chloride resin (1.4 mmol/g) and then stirred for 30 minutes. After stirring, the solvent was removed, and for the next reaction, Fmoc-L-6-aminohexanoic acid (14 mmol, 2 eq) and N,N'-diisopropylethylamine (28 mmol, 4 eq) were mixed with 40 mL of dichloromethane and then added to the resin. After stirring at room temperature for 2 hours, the reaction solution was removed. Subsequently, 50 mL each of dichloromethane and dimethylformamide was added three times to wash the remaining resin, thereby preparing Compound 1.

### Preparation of Compound 2

40 mL of a 20% (volume ratio) piperidine-containing dimethylformamide solution was added to the resin and stirred for 10 minutes at room temperature, and then the reaction solution was removed (repeated twice). Afterward, the remaining resin was washed by adding 20 mL each of dichloromethane and dimethylformamide three times. N-alpha-Fmoc-N-epsilon-[1-(4,4-dimethyl-2,6-dioxycyclohex-1-ylidine)ethyl]-L-lysine (14 mmol, 2 eq) which were used in the following reaction, hydroxybenzotriazole (28 mmol, 4 eq), and N,N'-diisopropylcarbodiimide (14 mmol, 2 eq) were added to the resin, and the resulting product was stirred for 2 hours at room temperature by using 50 mL of dimethylformamide as a solvent, following by removing the reaction solution. Subsequently, the above process was repeatedly performed in the same manner as described above by sequentially adding N-alpha-Fmoc-N-epsilon-[1-(4,4-dimethyl-2,6-dioxycyclohex-1-ylidine)ethyl]-L-lysine and 4,7,10,13,16,19,22,25-octaoxahexacosanoic acid. After reacting 4,7,10,13,16,19,22,25-octaoxahexacosanoic acid, 50 mL each of dichloromethane and dimethylformamide was added three times to wash the resin, thereby preparing Compound 2.

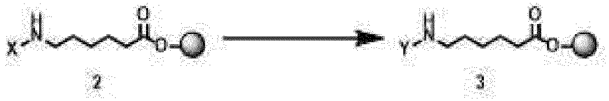

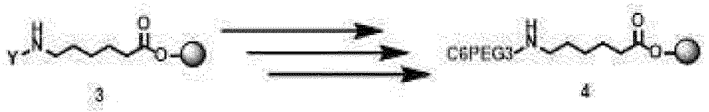

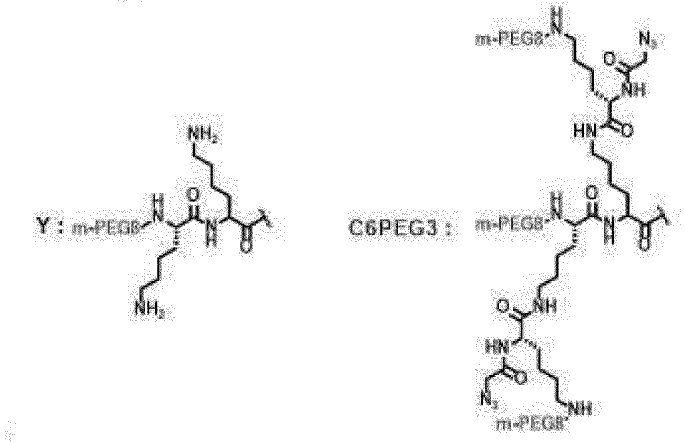

### Preparation of Compound 3

After 50 mL of 5% (volume ratio) hydrazine-containing dimethylformamide was added to the resin and stirred at room temperature for 10 minutes, the reaction solution was removed. Afterward, 50 mL each of dichloromethane and dimethylformamide was added three times to wash the resin, thereby preparing Compound 3.

### Preparation of Compound 4

N-alpha-Fmoc-N-epsilon-[1-(4,4-dimethyl-2,6-dioxycyclohex-1-ylidine)ethyl]-L-lysine (28 mmol, 4 eq), hydroxybenzotriazole (56 mmol, 8 eq), and N,N'-diisopropylcarbodiimide (28 mmol, 4 eq) were added to the resin and 50 mL of dimethylformamide as a solvent was added and stirred for 2 hours at room temperature, and then the reaction solution was removed. After the reaction, 40 mL of a 20% (volume ratio) piperidine-containing dimethylformamide solution was added to the resin and stirred for 10 minutes at room temperature, and then the reaction solution was removed (repeated twice). Subsequently, 50 mL each of dichloromethane and dimethylformamide was added three times to wash the resin. For the following reaction, after adding 2-azidoacetic acid (28 mmol, 2 eq), hydroxybenzotriazole (56 mmol, 4 eq) and N,N'-diisopropylcarbodiimide (28 mmol, 2 eq) were added to the resin and 50 mL of dimethylformamide as a solvent was added and stirred for 2 hours at room temperature, and the solution was removed. After the reaction, 50 mL each of dichloromethane and dimethylformamide was added three times to wash. The next process was performed in the same manner as the process of preparing Compound 3 above. 2,5,8,11,14,17,20,23-octaoxahexacosane-26-oic-acid (28 mmol, 2 eq), hydroxybenzotriazole (56 mmol, 4 eq), and N,N'-diisopropylcarbodiimide (28 mmol, 2 eq) were sequentially added and 50 mL of dimethylformamide as a solvent was added and stirred for 2 hours at room temperature, and then the solution was removed. After the reaction was completed, 50 mL each of dichloromethane and dimethylformamide was added three times to wash the resin, and the resulting resin was dried to prepare Compound 4.

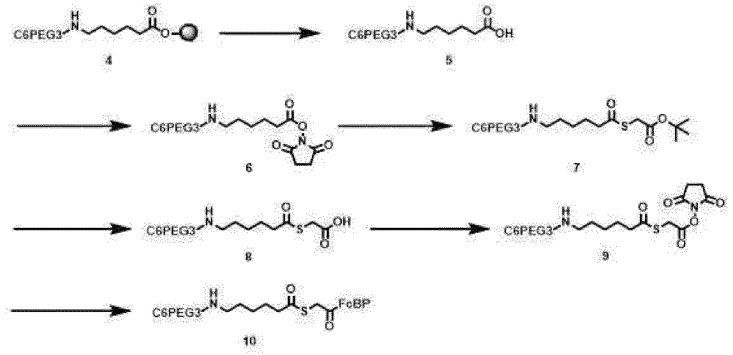

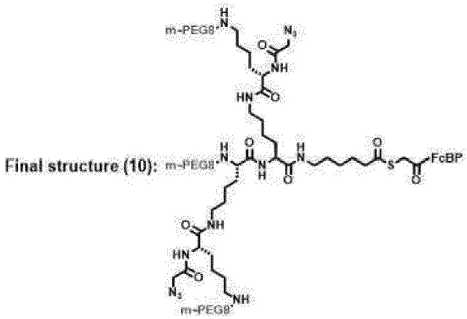

### Preparation of Compound 5

50 mL of trifluoroacetic acid (TFA), triisopropylsilane (TIS), and deionized water (DW) mixed in a volume ratio of 95 : 2.5 : 2.5 (TFA:TIS:DW) was added to the resin that had been processed until the preparation of Compound 4, and the resulting mixture was stirred at room temperature for 2 hours. After stirring, the solution and the resin were separated. 500 mL of 0 °C diethyl ether was added to the separated solution and mixed, and then left at 0 °C for 2 hours or more. After filtering the precipitated product, 50 mL of 0°C diethyl ether was added and then filtered (repeated twice). Subsequently, the resulting product was dried to prepare Compound 5 (5.11 mmol, 73%).

### Preparation of Compound 6

Compound 5 (0.996 g, 0.5 mmol) and tetramethyl-O-(N-succinimidyl)uronium tetrafluoroborate (1 mmol, 2 eq) were mixed with 5 mL of dimethylformamide, N,N'-diisopropylethylamine (1.5 mmol, 3 eq) was added, and the resulting mixture was stirred at room temperature for 1 hour. After the reaction was completed, trifluoroacetic acid was added until the pH of the solution reached 4.0. The reaction solution was purified by Prep-HPLC (C18) to obtain Compound 6 (0.375 mmol, 75%).

### Preparation of Compound 7

Compound 6 (0.375 mmol) and tert-butyl 2-mercaptoacetate (0.7 mmol, 2 eq) were added and mixed with 5 mL of dimethylformamide, N,N'-diisopropylethylamine (1.1 mmol, 3 eq) was added, and the resulting mixture was stirred at room temperature for 1 hour. After the reaction was completed, trifluoroacetic acid was added until the pH of the solution reached 4.0. The reaction solution was purified by Prep-HPLC (C18) and freeze-dried, thereby obtaining Compound 7 (0.3 mmol, 80%).

### Preparation of Compound 8

10 mL of trifluoroacetic acid and dichloromethane mixed in a 1:1 volume ratio was added to Compound 7 (0.375 mmol), and then stirred at room temperature for 1 hour. After the reaction was completed, the solution was removed using a vacuum concentrator, 10 mL of dichloromethane was added again to perform concentration under reduced pressure (repeated three times), thereby obtaining Compound 8. Compound 8 was dried and stored at -80 °C (0.297 mmol, 99%).

### Preparation of Compound 9

10 mL of a dimethylformamide solution was added to Compound 8 (0.375 mmol), and N,N'-diisopropylethylamine was added to adjust the pH to 8 to 9. After stirring the resulting mixture at room temperature for 10 minutes, tetramethyl-O-(N-succinimidyl)uronium tetrafluoroborate (0.7 mmol, 2 eq) and N,N'-diisopropylethylamine (0.375 mmol, 1 eq) were subsequently added and stirred at room temperature for 2 hours. After the reaction was completed, trifluoroacetic acid was added until the pH of the solution reached 4.0. The reaction solution was purified by Prep-HPLC (C18) and freeze-dried, thereby obtaining Compound 9 (0.173 mmol, 46%).

### Preparation of Compound 10

5 mL of a dimethylformamide solution was added to Compound 9 (0.173 mmol), an FcBP (Sequence: Ac-PEG8-DCAWH-(Dap)-GELVWCT-NH₂, Fc-binding peptide, L-form amino acid, disulfide bridge) (0.225 mmol, 1.2 eq) peptide composite that was synthesized by a solid phase peptide synthesis (SPPS) method was added, and N,N'-diisopropylethylamine (0.26 mmol, 1.5 eq) was added and allowed to react at room temperature for 1 hour. After the reaction was completed, the resulting product was purified by Prep-HPLC (C18) and freeze-dried, thereby obtaining Compound 10 (348 mg, 0.087 mmol, 50%).

### <Example 2-2> Preparation of linker (C6PEG4 linker)

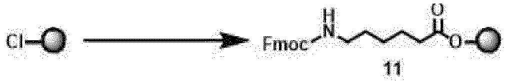

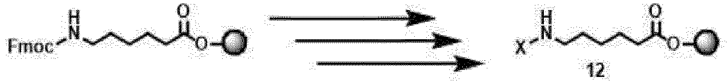

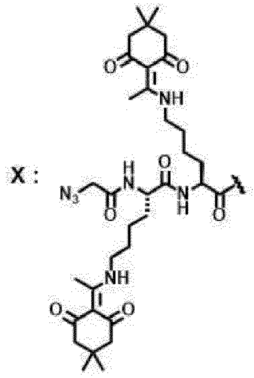

### Preparation of Compound 11

Compound 11 was synthesized by a solid phase synthesis method. 50 mL of dichloromethane was added to 2 g of 2-chlorotrityl chloride resin (1.4 mmol/g) and stirred for 30 minutes. After stirring, the solvent was removed, and for the next reaction, Fmoc-L-6-aminohexanoic acid (5.6 mmol, 2 eq) and N,N'-diisopropylethylamine (11.2 mmol, 4 eq) were mixed with 40 mL of dichloromethane and then added to the resin. After stirring at room temperature for 2 hours, the reaction solution was removed. Afterward, 50 mL each of dichloromethane and dimethylformamide was added three times to wash the remaining resin, thereby preparing Compound 11.

### Preparation of Compound 12

40 mL of a 20% (volume ratio) piperidine-containing dimethylformamide solution was added to the resin and stirred for 10 minutes at room temperature, and then the reaction solution was removed (repeated twice). Afterward, 20 mL each of dichloromethane and dimethylformamide were added three times to wash the remaining resin. N-alpha-Fmoc-N-epsilon-[1-(4,4-dimethyl-2,6-dioxycyclohex-1-ylidine)ethyl]-L-lysine (5.6 mmol, 2 eq) used in the next reaction, hydroxybenzotriazole (11.2 mmol, 4 eq), and N,N'-diisopropylcarbodiimide (5.6 mmol, 2 eq) were added to the resin and 50 mL of dimethylformamide as a solvent was added and stirred for 2 hours at room temperature, and then the reaction solution was removed. Afterward, this process was performed repeatedly in the same manner by sequentially adding N-alpha-Fmoc-N-epsilon-[1-(4,4-dimethyl-2,6-dioxycyclohex-1-ylidine)ethyl]-L-lysine and 2-azidoacetic acid. After the reaction of 2-azidoacetic acid, 50 mL each of dichloromethane and dimethylformamide was added three times to wash the resin, thereby preparing Compound 12.

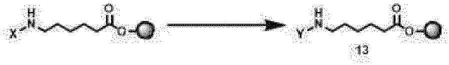

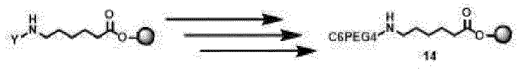

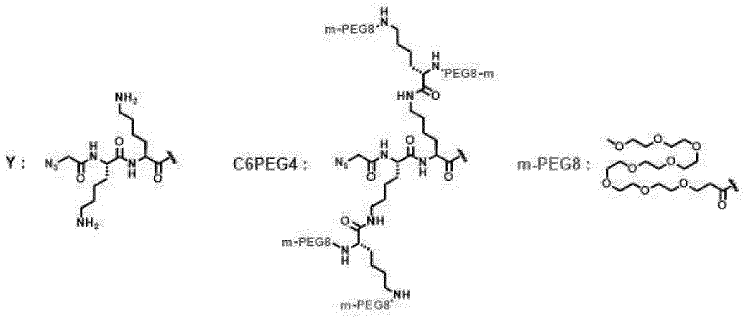

### Preparation of Compound 13

50 mL of 5% (volume ratio) hydrazine-containing dimethylformamide was added to the resin and stirred at room temperature for 10 minutes, and then the reaction solution was removed. Afterward, 50 mL each of dichloromethane and dimethylformamide was added three times to wash the resin, thereby preparing Compound 13.

### Preparation of Compound 14

N-alpha-Fmoc-N-epsilon-Fmoc-L-lysine (11.2 mmol, 4 eq), hydroxybenzotriazole (22.4 mmol, 8 eq), and N,N'-diisopropylcarbodiimide (11.2 mmol, 4 eq) were added to the resin and 50 mL of dimethylformamide as a solvent was added and stirred for 2 hours at room temperature, and then the reaction solution was removed. After the reaction, 40 mL of a 20% (volume ratio) piperidine-containing dimethylformamide solution was added to the resin and stirred for 10 minutes at room temperature, and then the solution was removed (repeated twice). Afterward, 50 mL each of dichloromethane and dimethylformamide was added three times to wash the resin. For the next process, 4,7,10,13,16,19,22,25-octaoxahexacosanoic acid (22.4 mmol, 8 eq) was added, and then hydroxybenzotriazole (44.8 mmol, 16 eq) and N,N'-diisopropylcarbodiimide (22.4 mmol, 8 eq) were added to the resin in the same manner as the above process, 50 mL of dimethylformamide as a solvent was added and stirred for 2 hours at room temperature, and then the reaction solution was removed. After the reaction, 50 mL each of dichloromethane and dimethylformamide was added three times to wash the remaining resin, and then the resulting resin was dried to prepare Compound 14.

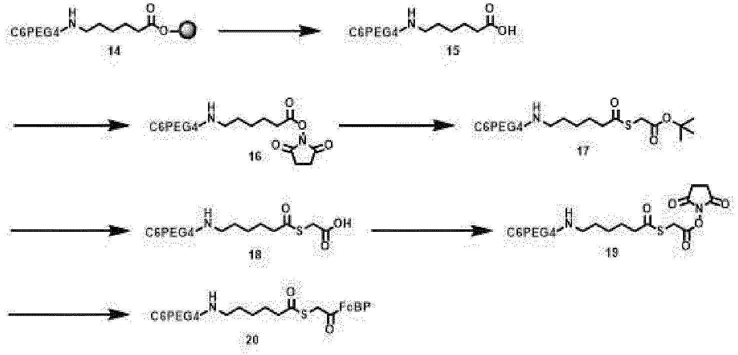

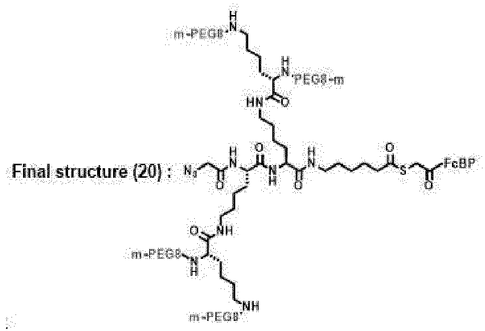

### Preparation of Compound 15

50 mL of trifluoroacetic acid (TFA), triisopropylsilane (TIS) and deionized water (DW) mixed in a volume ratio of 95 : 2.5 : 2.5 (TFA:TIS:DW) was added to the resin which had been processed until the preparation of Compound 14, and then stirred at room temperature for 2 hours. After stirring, the solution and the resin were separated. 500 mL of 0 °C diethyl ether was added to the separated solution and mixed, and then left for 2 or more hours. After filtering the precipitated product, 50 mL of 0 °C diethyl ether was added and then filtered again (repeated twice). Afterward, the resulting product was dried, thereby preparing Compound 15 (2.13 mmol, 76%).

### Preparation of Compound 16

Compound 15 (1 g, 0.433 mmol) and tetramethyl-O-(N-succinimidyl)uronium tetrafluoroborate (1 mmol, 2 eq) were added to 5 mL of dimethylformamide, and then N,N'-diisopropylethylamine (1.3 mmol, 3 eq) was added and stirred at room temperature for 1 hour. After the reaction was completed, trifluoroacetic acid was added until the pH of the solution reached 4.0. The reaction solution was purified by Prep-HPLC (C18) to obtain Compound 16 (0.303 mmol, 70%).

### Preparation of Compound 17

Compound 16 (0.303 mmol) and tert-butyl 2-mercaptoacetate (0.606 mmol, 2 eq) were added to 5 mL of dimethylformamide and then N,N'-diisopropylethylamine (0.909 mmol, 3 eq) was added and stirred at room temperature for 1 hour. After the reaction was completed, trifluoroacetic acid was added until the pH of the solution reached 4.0. The reaction solution was purified by Prep-HPLC (C18) and freeze-dried, thereby obtaining Compound 17 (0.242 mmol, 80%).

### Preparation of Compound 18

10 mL of trifluoroacetic acid and dichloromethane mixed in a 1:1 volume ratio was added to Compound 17 (0.242 mmol), and then stirred at room temperature for 1 hour. After the reaction was completed, the solution was removed using a vacuum concentrator, 10 mL of dichloromethane was added again to perform concentration under reduced pressure (repeated three times), thereby preparing Compound 18. Compound 18 was dried and stored at -80 °C.

### Preparation of Compound 19

10 mL of a dimethylformamide solution was added to Compound 18 (0.242 mmol), and N,N'-diisopropylethylamine was added to adjust the pH to 8 to 9. After stirring the resulting mixture at room temperature for 10 minutes, tetramethyl-O-(N-succinimidyl)uronium tetrafluoroborate (0.7 mmol, 2 eq) was added and N,N'-diisopropylethylamine (0.375 mmol, 1 eq) was added and stirred at room temperature for 2 hours. After the reaction was completed, trifluoroacetic acid was added until the pH of the solution reached 4.0. The reaction solution was purified by Prep-HPLC (C18) and freeze-dried, thereby obtaining Compound 19 (0.124 mmol, 51%).

### Preparation of Compound 20

5 mL of a dimethylformamide solution was added to Compound 19 (0.173 mmol) and then an FcBP (Sequence: Ac-PEG8-DCAWH-(Dap)-GELVWCT-NH₂, Fc-binding peptide, L-form amino acid, disulfide bridge) (0.225 mmol, 1.2 eq) peptide composite that was synthesized by a solid phase peptide synthesis (SPPS) method was added and N,N'-diisopropylethylamine (0.26 mmol, 1.5 eq) was added and allowed to react at room temperature for 1 hour. After the reaction was completed, the resulting product was purified by Prep-HPLC (C18) and freeze-dried, thereby obtaining Compound 20 (283 mg, 0.066 mmol, 53%).

## Claims

1. A conjugate represented by following Formula 1: 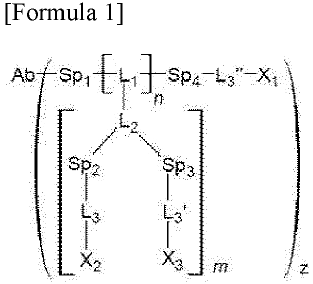 wherein,
Ab is a ligand,
Sp₁, Sp₂, Sp₃ and Sp₄ are each independently a spacer,
L₁ is a first linker unit,
L₂ is a second linker unit,
L₃, L₃' and L₃"are each independently selected from a third linker unit,
X₁, X₂, and X₃ are each independently selected from an active agent or a polyethylene glycol unit, herein, at least one or more of X₁, X₂, and X₃ are polyethylene glycol repeat unit,
z is an integer from 1 to 20,
n is an integer from 1 to 20,
m is an integer from 1 to 20,
herein, when n, m is 2 or more, L₁, L₂, L₃, L₃', Sp₂, Sp₃, X₂ and X₃ are each independently selected.

2. The conjugate according to claim 1, wherein
Ab is an antibody, an antibody fragment or an antigen-binding fragment thereof.

3. The conjugate according to claim 1, wherein
the spacer is one or more selected from a first spacer, a second spacer, a third spacer and a fourth spacer.

4. The conjugate according to claim 1, wherein
the spacer is two or more selected from a first spacer, a second spacer, a third spacer and a fourth spacer.

5. The conjugate according to claim 3, wherein
the first spacer, the second spacer, the third spacer and the fourth spacer are each independently selected from a group consisting of direct bond, substituted or unsubstituted C₁₋₅₀ alkylene, substituted or unsubstituted C₁₋₅₀ heteroalkylene, substituted or unsubstituted C₂₋₅₀ alkenylene, substituted or unsubstituted C₃₋₅₀ cycloalkylene, substituted or unsubstituted C₆₋₅₀ arylene, and substituted or unsubstituted C₆₋₅₀ heteroarylene.

6. The conjugate according to claim 3, wherein
one or more of the first spacer, the second spacer, the third spacer and the fourth spacer are unsubstituted C₁₋₁₀ heteroalkylene.

7. The conjugate according to claim 3,
wherein one or more of the first spacer, the second spacer, the third spacer and the fourth spacer are substituted C₁₋₂₀ heteroalkylene,
wherein when a group is substituted, the group is substituted with one selected from a group consisting of halo, =O, OH, NH₂, SH, NO₂, N₃, CN, OR₁, SR₁, OC(O)R₁, OC(O)NHR₁, OC(O)OR₁, CONHR₁, CON(R₁)₂, NHC(O)R₁, C(O)R₁, NHR₁, N(R₁)₂, C(O)R₁, OS(O)₂R₁, -OP(O)(OR₁)(OR₁), OP(O)(NHR₁)(NHR₁), and C₁₋₃ alkyl,
herein R₁ is selected from a group consisting of H, OH, C₁₋₅ alkyl, C₁₋₅ heteroalkyl, C₃₋₈ aryl and C₃₋₈ heteroaryl.

8. The conjugate according to claim 3,
wherein one or more of the first spacer, the second spacer, the third spacer and the fourth spacer are substituted C₆₋₅₀ heteroarylene,
wherein when a group is substituted, the group is substituted with one selected from halo, =O, OH, NH₂, SH, NO₂, N₃, CN, OR₁, SR₁, OC(O)R₁, OC(O)NHR₁, OC(O)OR₁, CONHR₁, CON(R₁)₂, NHC(O)R₁, C(O)R₁, NHR₁, N(R₁)₂, C(O)R₁, OS(O)₂R₁, -OP(O)(OR₁)(OR₁), OP(O)(NHR₁)(NHR₁), and C₁₋₃ alkylene,
herein R₁ is selected from a group consisting of H, OH, C₁₋₅ alkyl, C₁₋₅ heteroalkyl, C₃₋₈ aryl, C₃₋₈ heteroaryl.

9. The conjugate according to claim 3,
wherein one or more of the first spacer, the second spacer, and the third spacer are direct bond.

10. The conjugate according to claim 3,
wherein one or more of the first spacer, the second spacer, the third spacer, and the fourth spacer comprise terminal C(O).

11. The conjugate according to claim 3,
wherein the one or more of the first spacer, the second spacer, the third spacer and the fourth spacer are selected from a group consisting of -C(O)(CH₂)ₐ-, -NH(CH₂)ₐ-, -C(O)(CH₂)ₐC(O)-,-C(O)(CH₂)ₐNH-, -NH(CH₂)ₐNH-, -NHC(O)(CH₂)ₐC(O)-, -NHC(O)(CH₂)ₐNH-,-NHC(O)(CH₂)ₐC(O)NH-, and -NHC(O)(CH₂)ₐO(CH₂)_{b}-,
herein a and b are each independently an integer of 1 to 10.

12. The conjugate according to claim 3,
wherein one or more of the spacers are

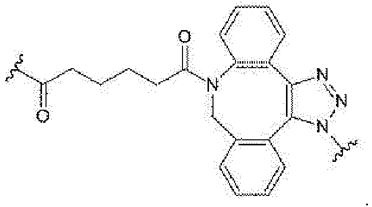

13. The conjugate according to claim 1,
wherein L₁ and L₂ are each independently selected from a group consisting of direct bond, amino acid, unnatural amino acid and derivatives thereof.

14. The conjugate according to claim 1,
wherein L₁ and L₂ are each independently selected from a group consisting of direct bond, alanine, β-alanine, γ-aminobutyric acid, arginine, asparagine, aspartic acid, γ-carboxyglutamic acid, citrulline, cysteine, glutamic acid, glutamine, glycine, histidine, isoleucine, leucine, lysine, methionine, norleucine, norvaline, ornithine, phenylalanine, proline, serine, threonine, tryptophan, tyrosine, valine and derivatives thereof.

15. The conjugate according to claim 1,
wherein L₁ and L₂ are each independently selected from a group consisting of direct bond, alanine, arginine, citrulline, isoleucine, leucine, lysine, phenylalanine, tryptophan, valine and derivatives thereof.

16. The conjugate according to claim 1,
wherein L₁ and L₂ are each independently selected from a group consisting of direct bond, alanine, citrulline, lysine, phenylalanine and valine.

17. The conjugate according to claim 1,
wherein one or more of L₁ and L₂ are cleavable by a tumor-related protease.

18. The conjugate according to claim 1,
wherein one or more of L₁ and L₂ are cleavable by a β-glucuronidase.

19. The conjugate according to claim 1,
wherein L₃, L₃' and L₃" are each independently direct bond or represented by following formula A: 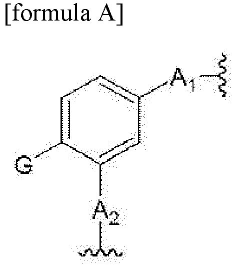 wherein,
G is glucuronic acid or its derivative,
A₁ and A₂ are each independently selected from a group consisting of direct bond, substituted or unsubstituted C₁₋₅₀ alkylene, substituted or unsubstituted C₁₋₅₀ heteroalkylene, substituted or unsubstituted C₂₋₅₀ alkenylene, substituted or unsubstituted C₃₋₅₀ cycloalkylene, substituted or unsubstituted C₆₋₅₀ arylene, and substituted or unsubstituted C₆₋₅₀ heteroarylene.

20. The conjugate according to claim 19,
wherein G is beta-glucuronic acid.

21. The conjugate according to claim 19,
wherein A₁ is bound to X₂, or X₃.

22. The conjugate according to claim 19,
wherein A₂ is bound to Sp₂, Sp₃ or Sp₄.

23. The conjugate according to claim 19,
wherein A₁ is selected from a group consisting of -(CH₂)ₐ₁OC(O)-(CH₂)_{b1}-, -(CH₂)ₐ₁C(O)-(CH₂)_{b1}-,-(CH₂)ₐ₁NH-(CH₂)_{b1}-, -(CH₂)ₐ₁C(O)NH-(CH₂)_{b1}- and -(CH₂)ₐ₁OC(O)NH-(CH₂)_{b1}-,
wherein a1 and b1 are each independently an integer of 0 to 10.

24. The conjugate according to claim 19,
wherein A₁ is -(CH₂)ₐ₁OC(O)-(CH₂)_{b1}-,
wherein a1 and b1 are each independently an integer of 0 to 5.

25. The conjugate according to claim 19,
wherein A₂ is represented by formula A-1:
[formula A-1] -A₂₁-A₂₂-A₂₃-
wherein A₂₁ and A₂₃ are each independently selected from a group consisting of -O-, -OCO-, -OCOO-, -OCONRₐ-, -NHRₐ-, -NRₐCO-, -NRₐCONR_{b}-, -NRₐCOO-, -NRₐCOR_{b}-, -NRₐCO-R_{b}-CO-, -NRₐCO-R_{b}-NH- and -S(O)-,
herein Rₐ and R_{b} are each independently H, or C₁₋₅ alkyl,
wherein A₂₂ is C₁₋₁₀ alkylene or C₂₋₁₀ alkenylene.

26. The conjugate according to claim 19,
wherein A₂ is -NHC(O)-(CH₂)₂-NH-.

27. The conjugate according to claim 1,
wherein at least two or more of X₁, X₂, and X₃ are polyethylene glycol unit.

28. The conjugate according to claim 1,
wherein the polyethylene glycol unit comprises -(CH₂CH₂O)- as a repeating unit.

29. The conjugate according to claim 1,
wherein the polyethylene glycol unit comprises -(CH₂CH₂O)ₓ- as a repeating unit, wherein x is an integer of 4 to 40.

30. The conjugate according to claim 1,
wherein the polyethylene glycol unit further comprises a binding part of -C(O)-.

31. The conjugate according to claim 1,
wherein the polyethylene glycol unit further comprises a terminal part of C₁₋₅ alkyl.

32. The conjugate according to claim 1,
wherein m is an integer of n or less, m repeating unit is each independently selected.

33. The conjugate according to claim 1,
wherein at least one or more of X₁, X₂, and X₃ are the active agent.

34. The conjugate according to claim 1,
wherein the active agent is selected from a group consisting of protein-targeting drugs, cell-targeting drug, DNA-targeting drug and RNA-targeting drug.

35. The conjugate according to claim 1,
wherein a linker-active agent except Ab of the formula 1 is 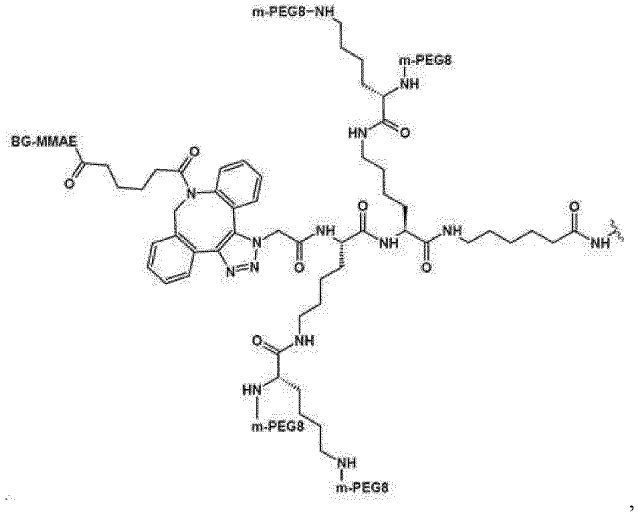 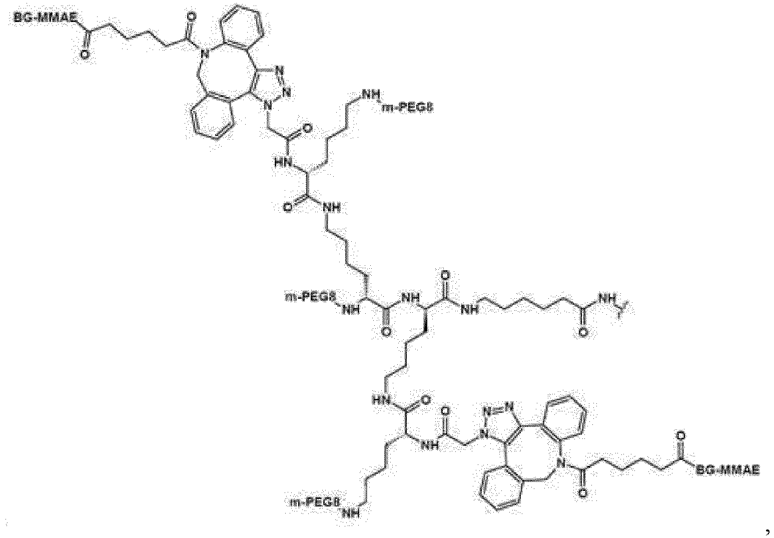 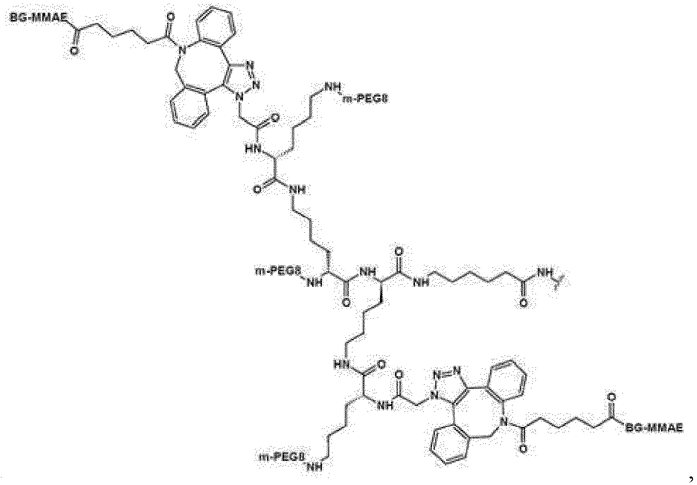 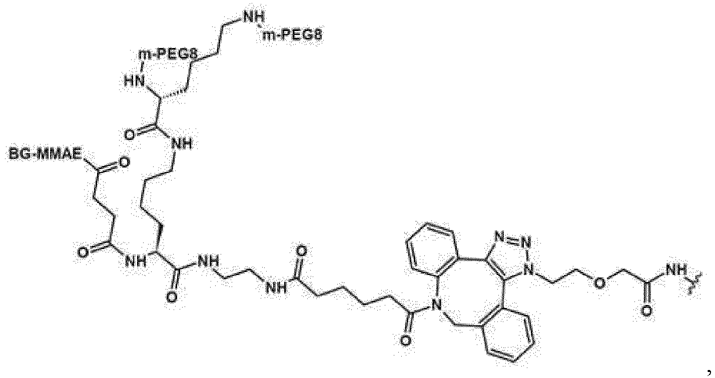 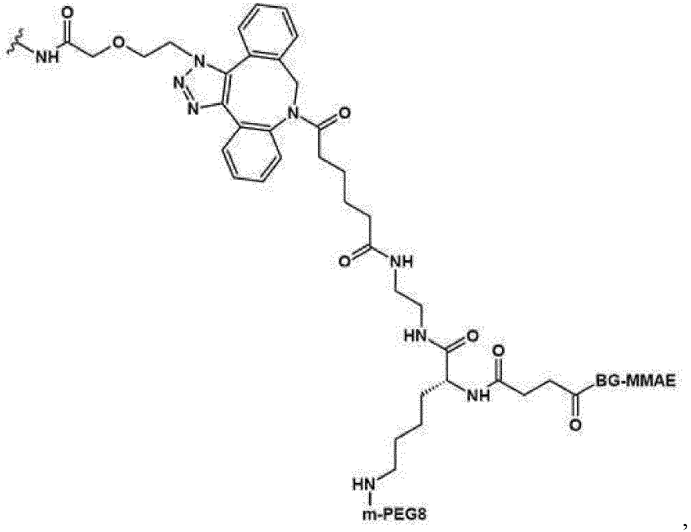 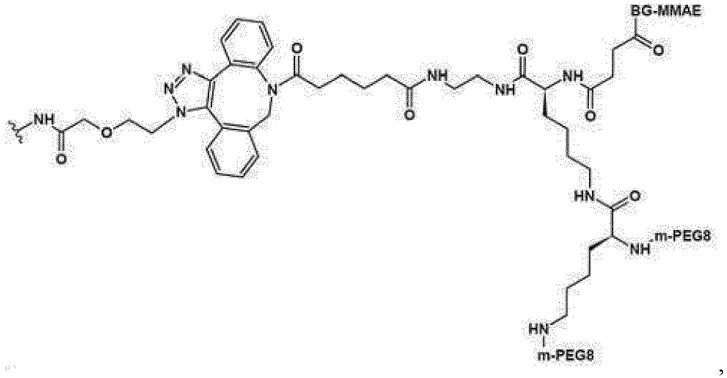 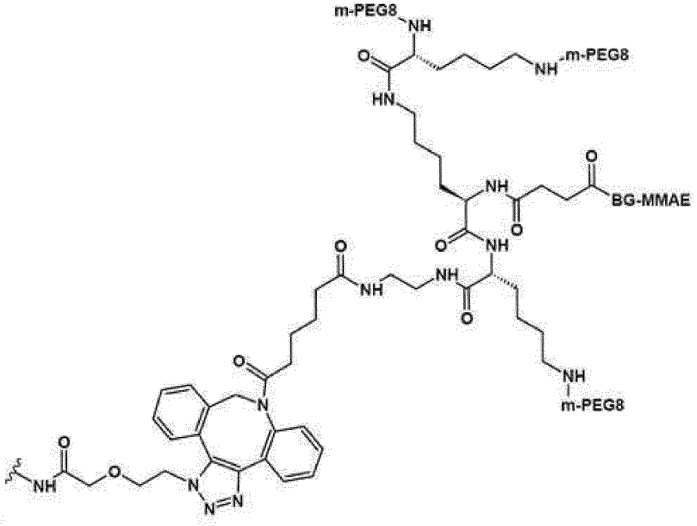 or 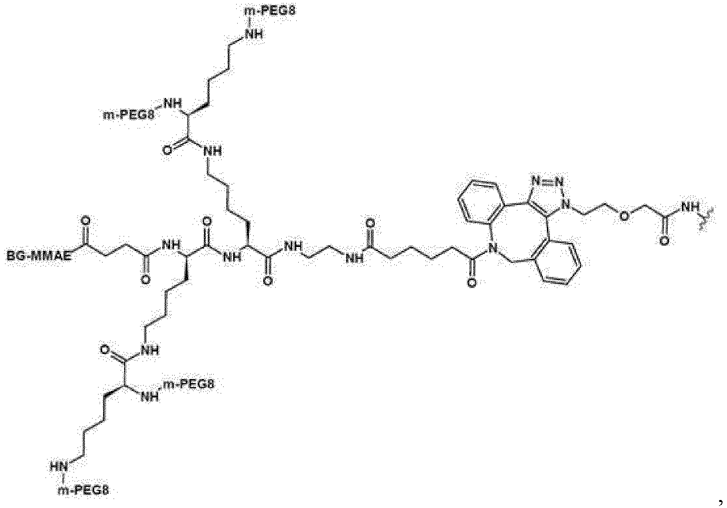 herein,
BG-MMAE is 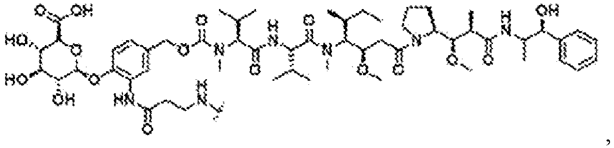
m-PEG8 is

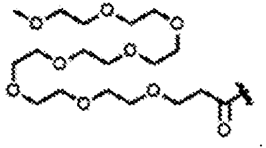

36. A pharmaceutical composition for preventing or treating a proliferative disease comprising a conjugate according to any one of claims 1 to 35.

37. A linker unit represented by following formula 1a: 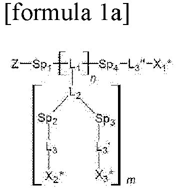 wherein,
Sp₁, Sp₂, Sp₃ and Sp₄ are each independently a spacer,
L₁ is a first linker unit,
L₂ is a second linker unit,
L₃, L₃' and L₃" are each independently selected from a third linker unit,
one or more of X₁*, X₂*, and X₃* are a polyethylene glycol unit,
one or more of X₁*, X₂*, and X₃* are active agent-binding moiety if are not polyethylene glycol unit,
n is an integer of 1 to 20,
m is an integer of 1 to 20,
herein when n or m is 2 or more, L₁, L₂, L₃, L₃', Sp₂, Sp₃, X₂* and X₃* are each independently selected,
Z is ligand-binding moiety.

38. The linker unit according to claim 37,
wherein the spacer is any one or more selected from the group consisting of a first spacer, a second spacer, a third spacer, and a fourth spacer.

39. The linker unit according to claim 37,
wherein the spacer is any two or more selected from the group consisting of a first spacer, a second spacer, a third spacer, and a fourth spacer.

40. The linker unit according to claim 38,
wherein the first spacer, the second spacer, the third spacer or the fourth spacer is independently selected from the group consisting of direct bond, substituted or unsubstituted C₁₋₅₀ alkylene, substituted or unsubstituted C₁₋₅₀ heteroalkylene, substituted or unsubstituted C₂₋₅₀ alkenylene, substituted or unsubstituted C₃₋₅₀ cycloalkylene, substituted or unsubstituted C₆₋₅₀ arylene, and substituted or unsubstituted C₆₋₅₀ heteroarylene.

41. The linker unit according to claim 38,
wherein one or more of the first spacer, the second spacer, the third spacer and the fourth spacer are unsubstituted C₁₋₁₀ heteroalkylene.

42. The linker unit according to claim 38,
one or more of the first spacer, the second spacer, the third spacer and the fourth spacer are substituted C₁₋₂₀ heteroalkylene,
wherein when a group is substituted, the group is substituted with one selected from the group consisting of halo, =O, OH, NH₂, SH, NO₂, N₃, CN, OR₁, SR₁, OC(O)R₁, OC(O)NHR₁, OC(O)OR₁, CONHR₁, CON(R₁)₂, NHC(O)R₁, C(O)R₁, NHR₁, N(R₁)₂, C(O)R₁, OS(O)₂R₁, -OP(O)(OR₁)(OR₁), OP(O)(NHR₁)(NHR₁), and C₁₋₃ alkyl,
herein, R₁ is selected from the group consisting of H, OH, C₁₋₅ alkyl, C₁₋₅ heteroalkyl, C₃₋₈ aryl and C₃₋₈ heteroaryl.

43. The linker unit according to claim 38,
wherein one or more of the first spacer, the second spacer, the third spacer and the fourth spacer are substituted C₆₋₅₀ heteroarylene,
wherein when a group is substituted, the group is substituted with one selected from the group consisting of halo, =O, OH, NH₂, SH, NO₂, N₃, CN, OR₁, SR₁, OC(O)R₁, OC(O)NHR₁, OC(O)OR₁, CONHR₁, CON(R₁)₂, NHC(O)R₁, C(O)R₁, NHR₁, N(R₁)₂, C(O)R₁, OS(O)₂R₁, -OP(O)(OR₁)(OR₁), OP(O)(NHR₁)(NHR₁), and C₁₋₃ alkylene,
herein R₁ is selected from the group consisting of H, OH, C₁₋₅ alkyl, C₁₋₅ heteroalkyl, C₃₋₈ aryl and C₃₋₈ heteroaryl.

44. The linker unit according to claim 38,
wherein one or more of the first spacer, the second spacer, the third spacer and the fourth spacer are direct bond.

45. The linker unit according to claim 38,
wherein one or more of the first spacer, the second spacer, the third spacer and the fourth spacer comprise terminal C(O).

46. The linker unit according to claim 38,
wherein one or more of the first spacer, the second spacer, the third spacer and the fourth spacer are selected from the group consisting of -C(O)(CH₂)ₐ-, -NH(CH₂)ₐ-, -C(O)(CH₂)ₐC(O)-, -C(O)(CH₂)ₐNH-, -NH(CH₂)ₐNH-, -NHC(O)(CH₂)ₐC(O)-, -NHC(O)(CH₂)ₐNH-, -NHC(O)(CH₂)ₐC(O)NH-, and-NHC(O)(CH₂)ₐO(CH₂)_{b}-,
herein a, b are each independently an integer of 1 to 10.

47. The linker unit according to claim 37,
wherein L₁ and L₂ are each independently selected from the group consisting of direct bond, amino acid, unnatural amino acid and derivatives thereof.

48. The linker unit according to claim 37,
wherein L₁ and L₂ are each independently selected from the group consisting of direct bond, alanine, β-alanine, γ-aminobutyric acid, arginine, asparagine, aspartic acid, γ- carboxyglutamic acid, citrulline, cysteine, glutamic acid, glutamine, glycine, histidine, isoleucine, leucine, lysine, methionine, norleucine, norvaline, ornithine, phenylalanine, proline, serine, threonine, tryptophan, tyrosine, valine and derivatives thereof.

49. The linker unit according to claim 37,
wherein L₁ and L₂ are each independently selected from the group consisting of direct bond, alanine, arginine, citrulline, isoleucine, leucine, lysine, phenylalanine, tryptophan, valine and derivatives thereof.

50. The linker unit according to claim 37,
wherein L₁ and L₂ are each independently selected from the group consisting of direct bond, alanine, citrulline, lysine, phenylalanine and valine.

51. The linker unit according to claim 37,
wherein one or more of L₁ and L₂ are cleavable by a tumor-related protease.

52. The linker unit according to claim 37,
wherein one or more of L₁ and L₂ are cleavable by a β-glucuronidase.

53. The linker unit according to claim 37,
wherein L₃, L₃' and L₃" are each independently direct bond, or represented by following formula A: 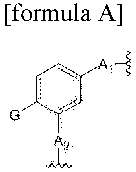 wherein,
G is glucuronic acid or its derivative,
A₁ and A₂ are each independently selected from the group consisting of direct bond, substituted or unsubstituted C₁₋₅₀ alkylene, substituted or unsubstituted C₁₋₅₀ heteroalkylene, substituted or unsubstituted C₂₋₅₀ alkenylene, substituted or unsubstituted C₃₋₅₀ cycloalkylene, substituted or unsubstituted C₆₋₅₀ arylene, and substituted or unsubstituted C₆₋₅₀ heteroarylene.

54. The linker unit according to claim 53,
wherein G is beta-glucuronic acid.

55. The linker unit according to claim 53,
wherein A₁ is bound to X₂, or X₃.

56. The linker unit according to claim 53,
wherein A₂ is bound to Sp₂, Sp₃ or Sp₄.

57. The linker unit according to claim 53,
wherein A₁ is selected from the group consisting of -(CH₂)ₐ₁OC(O)-(CH₂)_{b1}-, -(CH₂)ₐ₁C(O)-(CH₂)_{b1}-, -(CH₂)ₐ₁NH-(CH₂)_{b1}-, -(CH₂)ₐ₁C(O)NH-(CH₂)_{b1}- and -(CH₂)ₐ₁OC(O)NH-(CH₂)_{b1}-,
wherein a1 and b1 are each independently an integer of 0 to 10.

58. The linker unit according to claim 53,
wherein A₁ is -(CH₂)ₐ₁OC(O)-(CH₂)_{b1}-,
wherein a1 and b1 are each independently an integer of 0 to 5.

59. The linker unit according to claim 53,
wherein A₂ is represented by following formula A-1:
[formula A-1] -A₂₁-A₂₂-A₂₃-
wherein A₂₁ and A₂₃ are each independently selected from the group consisting of -O-, -OCO-,-OCOO-, -OCONRₐ-, -NHRₐ-, -NRₐCO-, -NRₐCONR_{b}-, -NRₐCOO-, -NRₐCOR_{b}-, -NRₐCO-R_{b}-CO-,-NRₐCO-R_{b}-NH- and -S(O)-,
herein Rₐ and R_{b} are each independently H, or C₁₋₅ alkyl,
wherein A₂₂ is C₁₋₁₀ alkylene or C₂₋₁₀ alkenylene.

60. The linker unit according to claim 53,
wherein A₂ is -NHC(O)-(CH₂)₂-NH-.

61. The linker unit according to claim 37,
wherein two or more of X₁*, X₂*, and X₃* are polyethylene glycol unit.

62. The linker unit according to claim 37,
wherein the polyethylene glycol unit comprises -(CH₂CH₂O)- as a repeating unit.

63. The linker unit according to claim 37,
wherein the polyethylene glycol unit comprises -(CH₂CH₂O)ₓ- as a repeating unit, wherein x is an integer of 4 to 40.

64. The linker unit according to claim 37,
wherein the polyethylene glycol unit further comprises a binding part of -C(O)-.

65. The linker unit according to claim 37,
wherein the polyethylene glycol unit further comprises a terminal part of C₁₋₅ alkyl.

66. The linker unit according to claim 37,
wherein m is an integer n or less, m repeating unit is each independently selected.

67. A method for treating a proliferative disease, comprising administering to a subject in need of treatment a therapeutic effective amount of a composition comprising a conjugate of any one of claims 1 to 35.

68. A use of a conjugate of any one of claims 1 to 35 for preparing a medicament for treating a proliferative disease.
